(19) Europäisches Patentamt
European Patent Office
Office européen des brevets

(11) EP 4 428 122 A1

(12) EUROPEAN PATENT APPLICATION

(43) Date of publication:
**11.09.2024 Bulletin 2024/37**

(21) Application number: **23160617.9**

(22) Date of filing: **07.03.2023**

(51) International Patent Classification (IPC):
***C07D 221/04*** (2006.01) ***C07C 229/14*** (2006.01)
***C07C 309/04*** (2006.01) ***C07D 311/58*** (2006.01)
***C07D 319/18*** (2006.01) ***A61K 31/198*** (2006.01)
***A61K 31/353*** (2006.01) ***A61K 31/357*** (2006.01)
***A61P 9/00*** (2006.01) ***A61P 13/12*** (2006.01)
***A61P 17/00*** (2006.01) ***A61P 25/00*** (2006.01)
***A61P 25/28*** (2006.01) ***A61P 27/02*** (2006.01)
***A61P 29/00*** (2006.01) ***A61P 35/00*** (2006.01)

(52) Cooperative Patent Classification (CPC):
**C07D 311/58; A61P 9/00; A61P 13/12; A61P 17/00; A61P 25/00; A61P 25/28; A61P 27/02; A61P 29/00; A61P 35/00; C07C 229/14; C07C 309/04; C07D 221/04; C07D 319/18;** C07C 2602/08; C07C 2602/10

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Vesper Bio Aps**
**1610 København V (DK)**

(72) Inventors:
- **Cases-Thomas, Manuel Javier**
  **2100 Copenhagen (DK)**
- **Little, Paul Brian**
  **2100 Copenhagen (DK)**
- **Kjølby, Mads Fuglsang**
  **2100 Copenhagen (DK)**
- **Nykjær, Anders**
  **2100 Copenhagen (DK)**

(74) Representative: **Gill Jennings & Every LLP**
**The Broadgate Tower**
**20 Primrose Street**
**London EC2A 2ES (GB)**

Remarks:
The complete document including Reference Table(s) and the Sequence Listing(s) can be downloaded from the EPO website

# (54) SORTILINE MODULATORS

(57) The present invention relates to compounds of Formula (I) or a pharmaceutically acceptable salt, solvate, hydrate, tautomer, optical isomer, N-oxide, and/or prodrug thereof. The present invention also relates to pharmaceutical compositions comprising the compounds of the invention, and to their use in the treatment or prevention of medical conditions in which modulation of sortilin is beneficial.

EP 4 428 122 A1

## Description

## FIELD OF THE INVENTION

The invention relates to compounds of Formula (I), which have surprisingly been found to modulate the activity of sortilin. The invention also relates to pharmaceutical compositions comprising these compounds, and to their use in the treatment or prevention of medical conditions in which modulation of sortilin activity is beneficial. In particular, the compounds of the invention may cross the blood brain barrier and may therefore be particularly useful in the treatment of diseases of the central nervous system. The compounds of the invention may also have an improved half-life *in vivo.*

## BACKGROUND

Sortilin is a Type I transmembrane protein that acts as a receptor of several ligands (Petersen et al., 1997). Sortilin is abundantly expressed in neurons and microglia of the nervous system, the inner ear, and in some peripheral tissues involved in metabolic control (Tauris et al., 2020; Goettsch et al., 2017; Willnow et al., 2011; Kjolby et al., 2010). Besides acting as a receptor involved in signaling, sortilin mediates sorting of select cargo between the cell surface trans-Golgi network, and endosomal pathway (Nykjaer & Willnow, 2012; Willnow, Petersen, & Nykjaer, 2008). Sortilin harbors a large extracellular domain denoted VPS10 that defines the family of receptors named sortilins or VS10p domain receptors. The VPS10P domain in sortilin is homologous to yeast VPS10P and is made up by a 10-bladed beta-propeller structure and a cysteine-rich 10CC module (Nykjaer & Willnow, 2012; Zheng, Brady, Meng, Mao, & Hu, 2011).

Sortilin binds multiple ligands, including pro-nerve growth factor (pro-NGF), pro-BDNF, pro-neurotrophin-3, neurotensin, and ApoB (Chen et al., 2005; Kjolby et al., 2010; Mazella et al., 1998; Nykjaer et al., 2004; Quistgaard et al., 2009; Yano, Torkin, Martin, Chao, & Teng, 2009). Furthermore, Sortilin binds progranulin (PGRN), a secreted protein involved in many cellular functions including securing lysosomal processes, anti-inflammatory responses, and neurotrophic stimulation (Galimberti, Fenoglio, & Scarpini, 2018). Sortilin targets PGRN for rapid endocytosis and degradation, and it is now well established that sortilin is the most important clearance receptor for PGRN (Hu et al., 2010). Thus, sortilin negatively regulates the extracellular levels of PGRN in the periphery as well as in the brain. Indeed, lack of or blocking the receptor increases plasma PGRN levels both in mice and humans (Carrasquillo et al., 2010; Gass, Prudencio, Stetler, & Petrucelli, 2012; Hu et al., 2010; Lee et al., 2014; Miyakawa et al., 2020; Pottier et al., 2018).

Frontotemporal dementia is a highly heritable dementia and haploinsufficiency of the PGRN gene accounts for up to 25% of all cases (Gijselinck, Van Broeckhoven, & Cruts, 2008). Patients with heterozygous loss-of-function mutations in PGRN have >50% reduced extracellular levels of the protein and will invariably develop FTD, making PGRN a causal gene for the disease (Baker et al., 2006; Carecchio et al., 2011; Cruts & Van Broeckhoven, 2008; Galimberti et al., 2010). In addition, PGRN mutant alleles have been identified in Alzheimer's (AD) patients (Brouwers et al., 2008; Sheng, Su, Xu, & Chen, 2014) and high levels of extracellular PGRN are protective in models of ALS, Parkinson's disease, stroke, arthritis, and atherosclerosis (Egashira et al., 2013; Laird et al., 2010; Martens et al., 2012; Tang et al., 2011; Tao, Ji, Wang, Liu, & Zhu, 2012; Van Kampen, Baranowski, & Kay, 2014).

Sortilin is, however, not required for PGRN to elicit its functions. Hence, neurons devoid in sortilin expression are equally responsive to PGRN-induced neuronal outgrowth (De Muynck et al., 2013; Gass, Lee, et al., 2012). Further, PGRN is successfully delivered to neuronal lysosomes in sortilin-deficient cells, suggesting the existence of alternative trafficking pathways. Indeed, PGRN can bind to the lysosomal protein, prosaposin (PSAP). When PSAP binds to its cognate receptors, the cation-independent mannose-6-phosphate receptor and LRP1, it brings along PGRN to the lysosomes (Zhou et al., 2015). Finally, in a phase II clinical trial with a monoclonal anti-sortilin antibody, markers for lysosomal integrity were normal (NCT03987295).

The functional PGRN receptor remains to be identified. However, studies suggest that PGRN promotes neuronal survival, reduces inflammation and increases Aβ endocytosis by microglia (Martens et al., 2012; Pickford et al., 2011; Yin et al., 2010).

Binding of PGRN to sortilin requires the three amino acids in the C-terminal of PGRN (QLL in human, PLL in mouse), and a peptide derived from the last 24 amino acids of PGRN binds with similar affinity as the full-length protein (Zheng et al., 2011). It was proposed that this mode of binding is structurally similar to Neurotensin binding (Zheng et al., 2011), i.e. binding in the NTIS1 binding site of sortilin. There has been a successful small molecule screen that identified a blocker of Neurotensin to sortilin binding done in collaboration with Aarhus University (Andersen et al., 2014; Schroder et al., 2014).

Sortilin exists as a full-length and sorting competent receptor but is also capable of forming multimeric signalling receptor-ligand. Portions of sortilin can also be liberated from the plasma membrane to scavenge ligands (NT in pain) and control the activity of ligands. For example, sortilin is involved in synaptic plasticity by controlling the conversion rate of pro-BDNF into BDNF. This may also apply to other proneurotrophins.

Finally, the propeptide of sortilin, also named spadin, that is a ligand of the receptor has been demonstrated

to control the activity of the membrane transporter TREK-1, which is a target for major depression, among other diseases. Structurally, sortilin has an amino acid sequence according to SEQ ID NO: 1 and comprises a signal peptide, a propeptide, the Vps10p domain, a 10cc domain (10CCa + 10CCb), a transmembrane domain and a cytoplasmic tail. The luminal domain of sortilin has 6 potential N-linked glycosylation sites, whilst the cytoplasmic tail enables for the recruitment of various adapter proteins.

Sortilin binds to a vast number of ligands and membrane receptors and as a result engages in functions known to be important in cellular signalling and sorting. For example, sortilin is involved in signalling by proneurotrophins: the proforms of nerve growth factor (pro-NGF), brain derived neurotrophic factor (pro-BDNF), and neurotrophin-3 (proNT3), respectively. In complex with the protein p75NTR (p75 neurotrophin receptor), sortilin has been reported to form the receptor for proneurotrophin-mediated apoptotic effects leading to degeneration and cell death in cellular and animal models (Jansen et al., 2007; Tenk et al., 2005; Nykjaer et al., 2004).

Previous work has suggested a role for sortilin in cellular sorting and signalling associated with diseases such as diabetes and obesity (Huang et al., 2013). Sortilin facilitates translocation of GLUT4 to the plasma membrane and rescues it from degradation in the lysosomes (Pan et al., 2017). Sortilin levels have been shown to be modulated by the level of inflammation associated with these diseases. The pro-inflammatory cytokine, TNF$\alpha$, reduces both mRNA levels and protein levels of sortilin in cultured mouse and human adipocytes, as well as *in vivo* when injected into mice (Kaddai et al., 2009). Sortilin can also influence cytokine secretion: targeting sortilin in immune cells has been proposed to attenuate inflammation and reduce atherosclerosis disease progression (Mortensen et al., 2014). Additionally, US 2016/0331746 describes various scaffolds of small molecules capable of binding to the active site of sortilin. Sortilin is involved in the regulation of glucose uptake (Shi & Kandror. 2005) and the development of lipid disorder diseases (Gao et al., 2017).

Further, plasma sortilin levels have been reported to be a potential biomarker for identifying patients with either coronary heart disease or diabetes mellitus (Oh et al., 2017; Møller et al., 2021). Patients that showed increased sortilin levels within their plasma, and therefore identifiable as suffering from the above conditions, also displayed enhanced glucose levels suggesting sortilin as a therapeutic target for treating these conditions. Soluble sortilin is also proposed as a treatment for type II diabetes (WO2021116290 A1, 2021).

TAR DNA-binding protein 43 (TDP-43) has been implicated in various neurodegenerative diseases. For example, TDP-43 inclusion bodies have been found in cases of amyotrophic lateral sclerosis (ALS), frontotemporal lobar degeneration (FTLD), and Alzheimer's disease (AD) (Meneses et al., 2021).

TDP-43 regulates the splicing of several gene products, including Sortilin. In humans, the splicing involves inclusion of a cryptic exon 17b (between exon 17 and 18), which introduces a stopcodon in the stalk, potentially generating a non-membrane bound fragment (Prudencio et al., 2012). Furthermore, it has been shown that PGRN can reduce levels of insoluble TDP-43 and slow down axonal degeneration (Beel et al., 2018). Sortilin inhibition increases PGRN levels and is therefore beneficial in the treatment of neurodegenerative diseases in which TDP-43 is implicated.

Sortilin has been linked to various conditions affecting the central nervous system (CNS). Some studies suggest a role of circulating sortilin in patients with psychiatric disorders such as depression, which may relate to altered activity of neurotrophic factors (Buttenshon et al., 2015); and it has also been reported that sortilin plays a role in brain aging, Alzheimer's Disease and frontotemporal dementia (Xu et al., 2019). However, delivering therapeutic agents that are capable of crossing the blood-brain barrier to the CNS presents a major challenge.

The blood-brain barrier is a highly selective semipermeable border of endothelial cells that prevents solutes in the circulating blood from non-selectively crossing into the extracellular fluid of the CNS where neurons reside. Therapy of neurological diseases is therefore limited due to the restricted penetration of therapeutic agents across the blood-brain barrier.

Therapeutic agents for treating CNS diseases therefore must be capable of crossing the blood-brain barrier. In addition to this, they must also have a sufficient unbound drug concentration in the brain, as the free drug hypothesis states that only unbound compound is able to interact with and elicit a pharmacological effect.

To determine the unbound fraction of a test compound ($F_{ub}$), sample supernatants may be analysed by methods such as liquid chromatography with tandem mass spectrometry (LC-MS/MS). The unbound fraction may then be calculated from the peak area ratios obtained for each matrix according to the following formula:

$$F_{ub} = C_{PBS} / C_{plasma}$$

where $C_{PBS}$ and $C_{plasma}$ are the analyte concentrations in PBS (receiver) and plasma (donor), respectively.

Recovery samples may be prepared in each condition but without dialysis, and may be used for evaluation of recovery from dialysis experiments using the following formula:

$$\% \text{ Recovery} = 100 \times (V_{PBS} \times C_{PBS} + V_{plasma} \times C_{plasma}) / V_{plasma} \times C_{recovery}$$

where $V_{PBS}$ is the volume on the receiver side (PBS) and $V_{plasma}$ is the volume on donor side (plasma) of the dialysis device. $C_{recovery}$ is the analyte concentration measured from the recovery sample. Compounds such as propranolol or fluoxetine, may be included in experiments as controls.

The unbound fraction in brain ($F_{ub, brain}$) may be calculated from the measured value in brain homogenate ($F_{ub, meas}$), taking into account the dilution factor used in preparing the brain homogenate:

$$F_{ub,\,brain} = \frac{1/D}{\left(\frac{1}{F_{ub,\,meas}}\right) - 1 + \left(\frac{1}{D}\right)}$$

where D = dilution factor.

The brain/plasma unbound partition coefficient ($K_{puu}$) may be determined as a ratio between the free compound concentrations in plasma and brain:

$$K_{puu} = \frac{C_{ub,brain}}{C_{ub,plasma}}$$

Where $C_{u,brain}$ = unbound concentration in brain (C x $F_{ub, brain}$); wherein

C = concentration at steady state; and
$C_{ub,plasma}$ = unbound concentration in plasma (C x $F_{ub}$).

Alternatively a ratio of AUCs can be used to determine $K_{puu}$. The compound of interest being dosed to a relevant species of interest at a known concentration by oral or intravenous route. Timecourses of concentration of compound in the plasma and cerebral spinal fluid (CSF) are measured. The plasma concentration is corrected to account for unbound fraction of compound. Areas under the CSF concentration curve and free fraction in plasma concentration curve are calculated by known methods and a ratio is determined to give:

$$K_{puu} = \text{AUC0-infcsf} / (\text{AUC0-infplasma} \times (\%\text{Fuplasma}/100))$$

For the treatment of CNS diseases, it is desirable for the $K_{puu}$ to have the highest possible value, above 0. A value around 1 indicating that the free fraction compound freely permeates the blood brain barrier; a value above 1 suggesting that an active influx transport mechanism at the blood brain barrier is involved; and less than 1, which indicates that the free fraction compound is either poorly permeable or is recognized by an active efflux mechanism, reducing the exposure in the CNS while passing back through the blood-brain barrier to plasma or the CSF. A $K_{puu}$ value of 0 or close to 0, indicates a poorly permeable compound or a highly active efflux mechanism that in any case will make highly improbable to reach a meaningful exposure in the CNS of the desired active species.

In addition to effectively targeting and binding to sortilin, compounds useful for modulating sortilin activity in a subject preferably have optimised pharmacokinetics. For example, this may equate to a prolonged half-life *in vivo* or improved delivery to target tissues. Improved persistence of such compounds in a subject is an important clinical parameter. This determines the required dosages and frequency of dosing of the compounds, as well as formulation requirements needed to achieve the desired therapeutic effect upon administration.

An increase in the *in-vivo* half-life ensures that the compounds remain therapeutically effective in the subject for as long as possible before being metabolised or excreted by the body, which permits administration of lower effective dosages and/or less frequent dosing of the therapeutic compounds. Accordingly, such compounds, with increased half-life are of significant pharmaceutical importance.

The current inventors have devised compounds which can modulate sortilin activity, as disclosed in European Application No. EP22193792.3, the entire contents of which are hereby incorporated by reference.

In view of the above, there is an unmet need for further compounds that may be used in the treatment and prevention of medical conditions in which modulation of sortilin is beneficial. In particular, there is an unmet need for further sortilin modulators that are capable of crossing the blood brain barrier and are therefore useful in the treatment of diseases of the central nervous system. Furthermore, there is a need for sortilin modulators that have an improved

half-life *in vivo.*

## DISCLOSURE OF THE INVENTION

Surprisingly, it has been found that compounds of Formula (I) modulate the activity of sortilin and are therefore useful in the treatment or prevention of conditions in which modulation of sortilin is beneficial. Furthermore, the compounds may cross the blood brain barrier and may therefore be particularly useful in the treatment of diseases of the central nervous system.

Without wishing to be bound by theory, it is believed that the backbone of the compounds of Formula (I) is not recognised by peptidases as a natural amino acid residue due to quaternary carbon adjacent to the carboxylic acid group. Accordingly, it is expected that the unnatural amino acid residue will resist degradation for longer and metabolise at a slower rate. It is therefore expected that the compounds of the invention will exhibit an improved half-life *in vivo,* for example compared to the compounds disclosed in EP22193792.3.

In a first aspect of the invention, there is provided a compound of Formula (I):

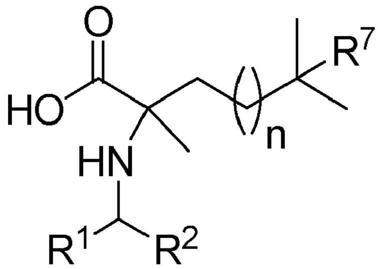

(I)

or a pharmaceutically acceptable salt, solvate, hydrate, tautomer, optical isomer, N-oxide, and/or prodrug thereof; wherein

$R^1$ is

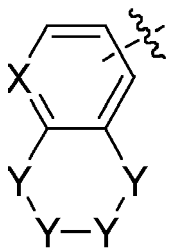 or 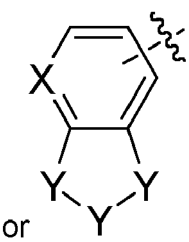 ;

X is $CR^3$ or N, wherein $R^3$ is selected from the group consisting of H, halo, and $C_1$-$C_4$ alkyl;

each Y is independently $CHR^4$, $NR^5$, $CR^6R^6$, or O, wherein $R^4$ is independently selected from the group consisting of H, halo, oxo, and $C_1$-$C_4$ alkyl; $R^5$ is independently selected from the group consisting of H, halo, $C_1$-$C_4$ alkyl, $C_1$-$C_4$ hydroxyalkyl, -($C_1$-$C_4$ alkyl)-O-($C_1$-$C_4$ alkyl), -C(O)-($C_1$-$C_4$ alkyl), and -C(O)O-($C_1$-$C_4$ alkyl); and $R^6$ is independently selected from the group consisting of halo and $C_1$-$C_4$ alkyl;

$R^2$ is selected from the group consisting of: H, $C_1$-$C_4$ alkyl, $C_1$-$C_4$ hydroxyalkyl and $C_1$-$C_4$ haloalkyl;

$R^7$ is H or $-CH_3$;

n = 0 or 1.

Surprisingly, the applicant has found that when $R^7$ is $-CH_3$ and n is 1, the compound of Formula (I) exhibits improved binding to sortilin. In a preferred aspect of the invention, the compound of Formula (I) therefore has the following

formula:

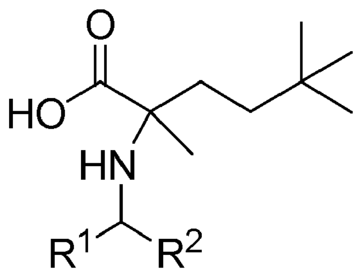

In the compounds of the invention, $R^2$ is preferably selected from the group consisting of H, $CH_3$, $CH_2F$, $CHF_2$, and $CF_3$, more preferably $R^2$ is selected from the group consisting of H, $CH_3$, $CHF_2$, and $CF_3$, more preferably H.

$R^3$ is preferably H or $C_1$-$C_4$ alkyl, more preferably H or methyl.

Each $R^4$ is preferably independently H or oxo.

Each $R^5$ is preferably independently H, $C_1$-$C_4$ alkyl, or -C(O)O-($C_1$-$C_4$ alkyl), more preferably H, methyl, or -C(O)O-(tert-butyl), more preferably H or methyl, most preferably H.

As defined above, $R^1$ is a partially saturated fused bicyclic ring system. The ring system may be attached to the remainder of the molecule at any suitable position on the aromatic ring. However, in a particularly preferred aspect of the invention, $R^1$ is selected from one of the following groups:

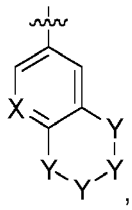 , 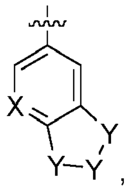 , 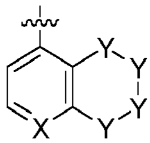 , and 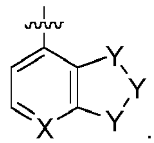 .

The partially saturated ring of the $R^1$ group may comprise one or more heteroatoms. However, it is preferred that no more than two atoms in the partially saturated ring are heteroatoms. Therefore, no more than one or two Y are preferably $NR^5$ or O and the remaining Y are preferably $CHR^4$ or $CR^6R^6$.

The carbon atoms in the partially saturated ring of the $R^1$ group may be substituted with one or two substituents as defined above. However, it is preferred that any carbon atoms in the partially saturated ring are substituted with no more than 1 substituent. Therefore, if a ring atom in the partially saturated ring is carbon, it is preferably $CHR^4$.

Therefore, in a particularly preferred aspect of the invention, no more than two Y are $NR^5$ or O and the remaining Y are $CHR^4$.

Preferable examples of the $R^1$ group include:

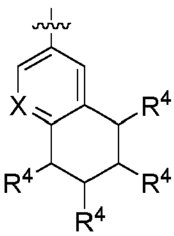 , 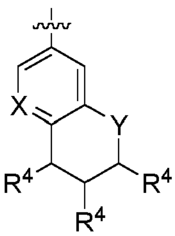 , 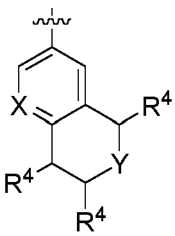 , 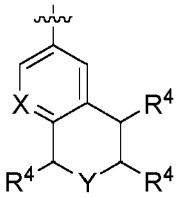 , 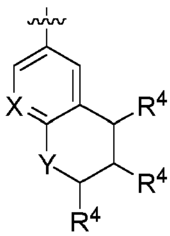 , 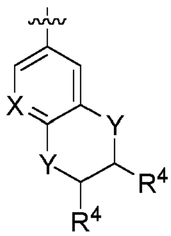 ,

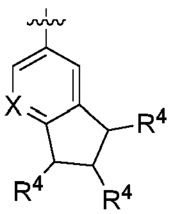 , 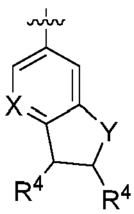 , 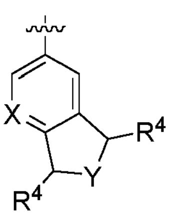 , 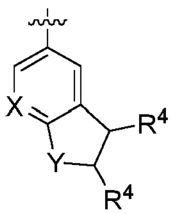 , 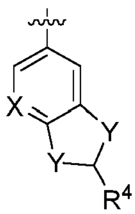 , 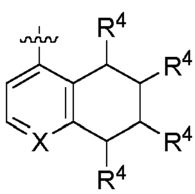 ,

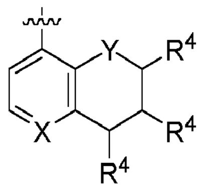, 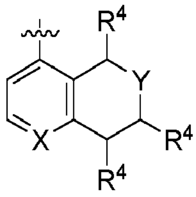, 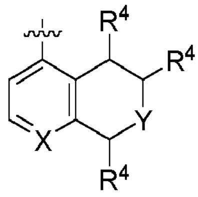, 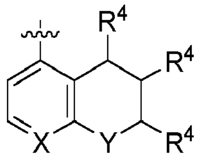, 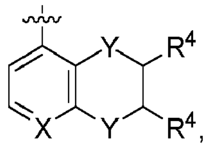

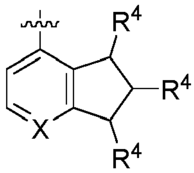, 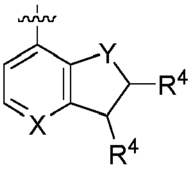, 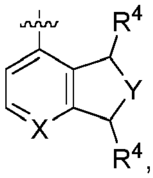, 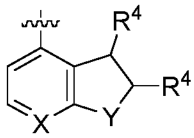, and 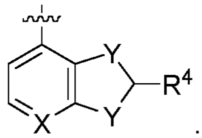.

In the preferred $R^1$ groups above, each Y is independently $NR^5$ or O. However, when the partially saturated ring comprises two Y atoms, it is preferred that one Y is O and the other Y is O or N.

It is also preferred that only one of the carbon atoms in the partially saturated ring is substituted with a substituent. Therefore, one $R^4$ group is preferably selected from the group consisting of H, halo, oxo, and $C_1$-$C_4$ alkyl and any other $R^4$ groups are all H. More preferably, one $R^4$ group is H or oxo and any other $R^4$ groups are all H.

In the compounds of formula (I), when the $R^1$ group is attached to the remainder of the molecule at the position ortho- to the saturated ring, X may be CH and Y may be O.

In a more preferred aspect of the invention, $R^1$ is selected from one of the following groups:

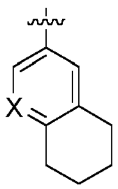, 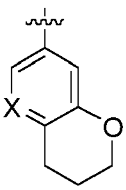, 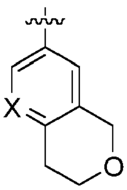, 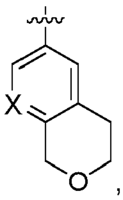, 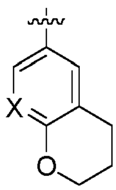, 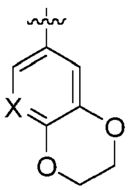, 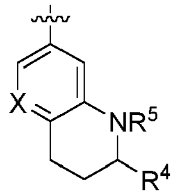,

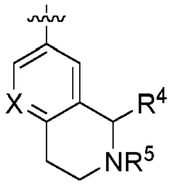, 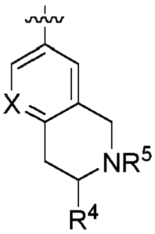, 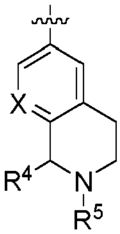, 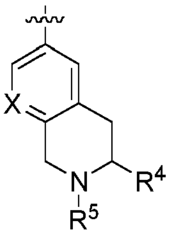, 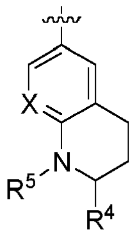, 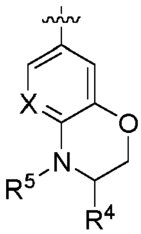,

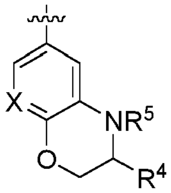, 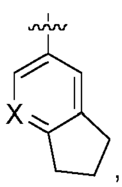, 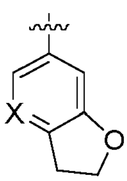, 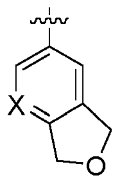, 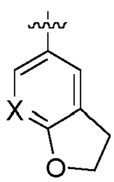, 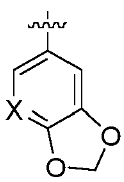, 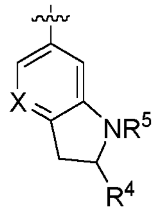,

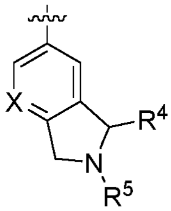, 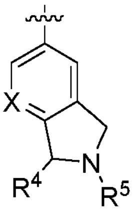, 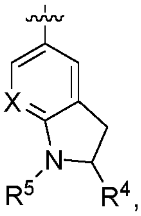, 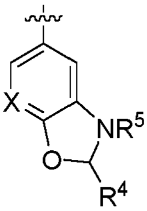, 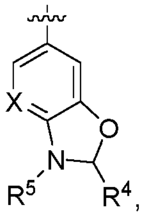, 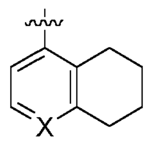, 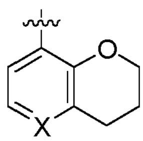,

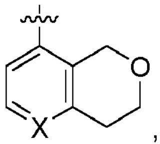, 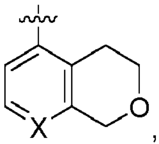, 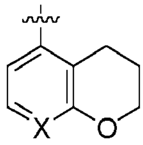, 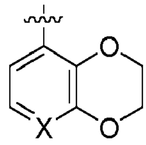, 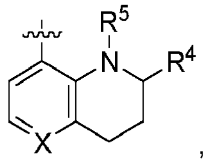, 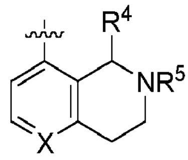,

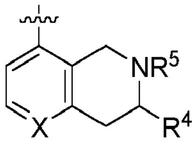, 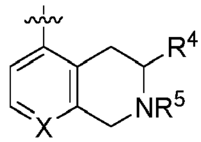, 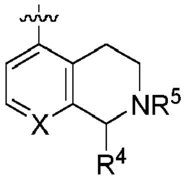, 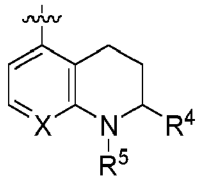, 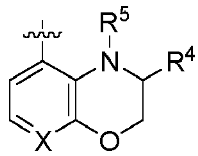,

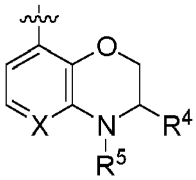, 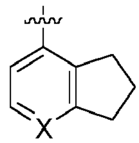, 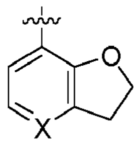, 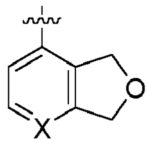, 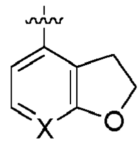, 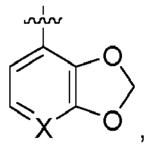,

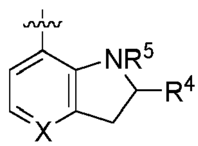, 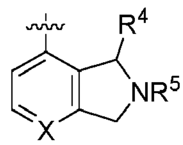, 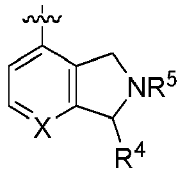, 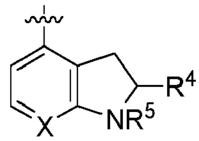, 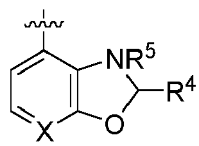,

and

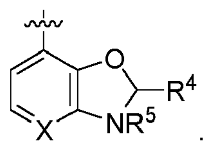.

More preferably, $R^1$ is selected from one of the following groups:

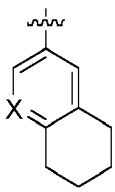, 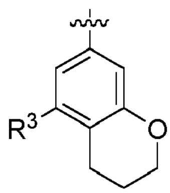, 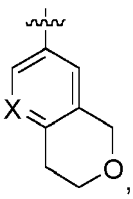, 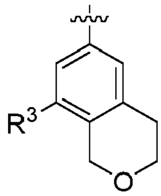, 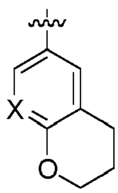, 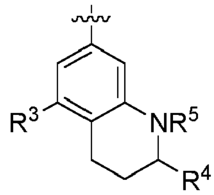,

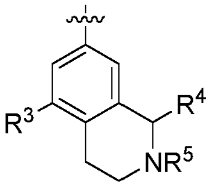, 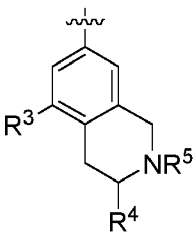, 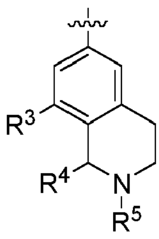, 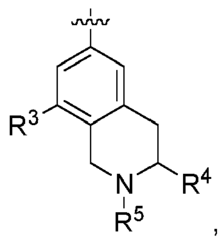, 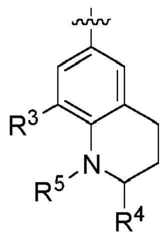, 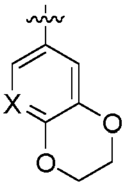

, 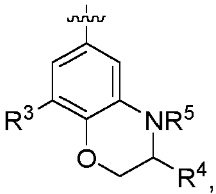, 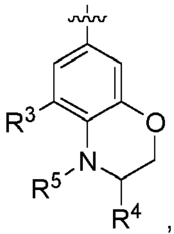, 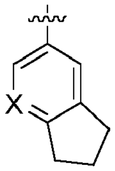, 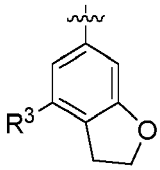, 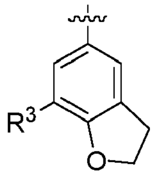, 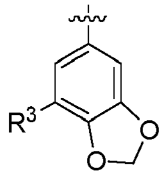,

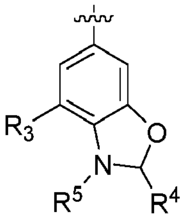, 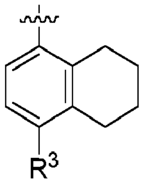, 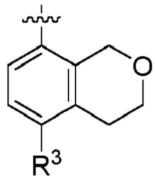, 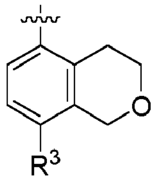, 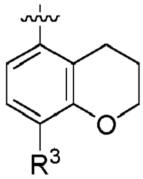, 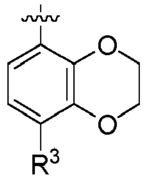,

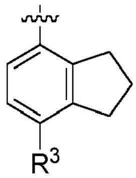, 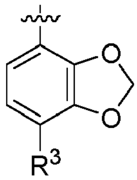, and 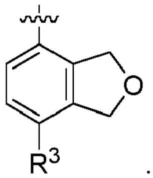.

Most preferably, $R^1$ is selected from one of the following groups:

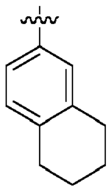, 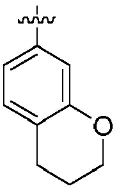, 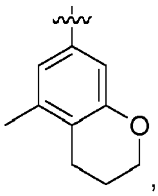, 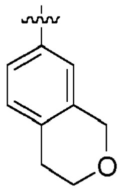, 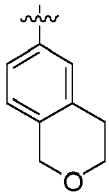, 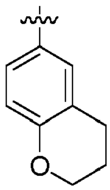, 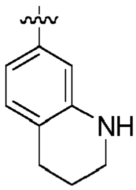, 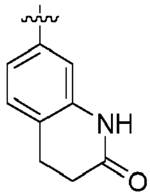,

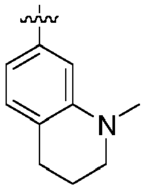, 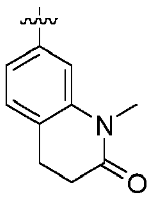, 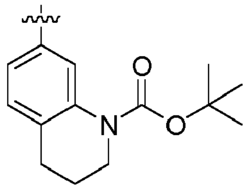, 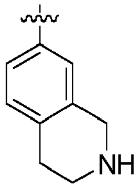, 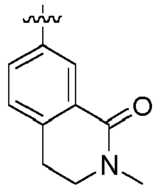, 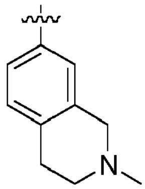,

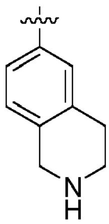, 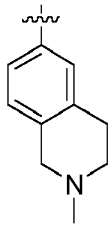, 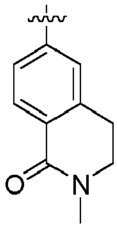, 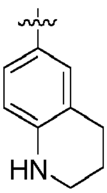, 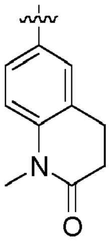, 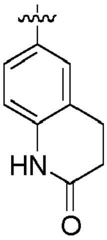, 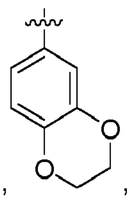, 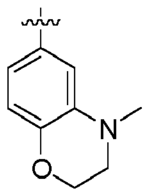,

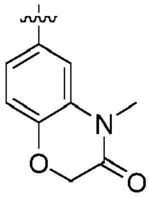, 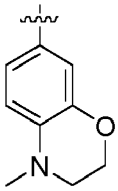, 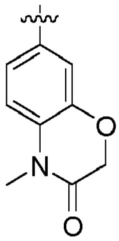, 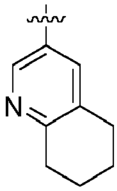, 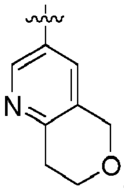, 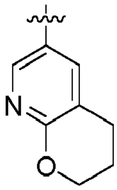, 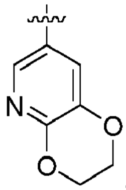, 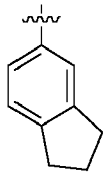,

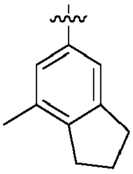, 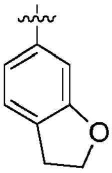, 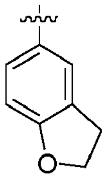, 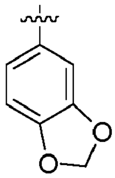, 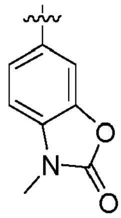, 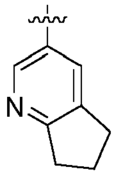, 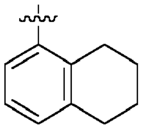, 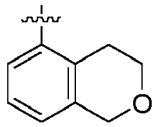,

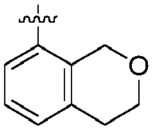, 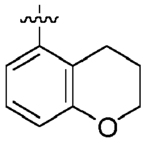, 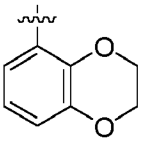, 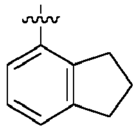, 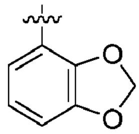, and 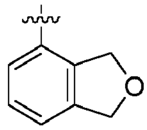,

preferably $R^1$ is selected from one of the following groups:

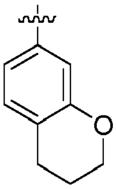, 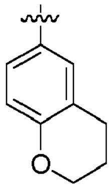, 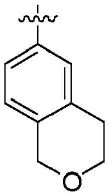, 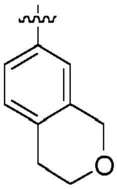, 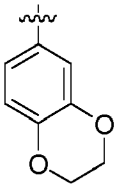, 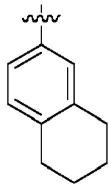, 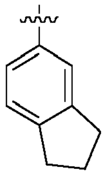, and 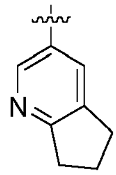.

Particular compounds of the invention are those listed below.

2,5,5-trimethyl-2-{[(5,6,7,8-tetrahydro-2-naphthyl)methyl]amino}hexanoic acid;

(S)-2,5,5-trimethyl-2-{[(5,6,7,8-tetrahydro-2-naphthyl)methyl]amino}hexanoic acid;

(R)-2,5,5-trimethyl-2-{[(5,6,7,8-tetrahydro-2-naphthyl)methyl]amino}hexanoic acid;

2-{[(5-indanyl)methyl]amino}-2,5,5-trimethylhexanoic acid;

2-{[(7-chromanyl)methyl]amino}-2,5,5-trimethylhexanoic acid;

2-{[(6-chromanyl)methyl]amino}-2,5,5-trimethylhexanoic acid;

2-{[(6-isochromanyl)methyl]amino}-2,5,5-trimethylhexanoic acid;

2-{[(7-isochromanyl)methyl]amino}-2,5,5-trimethylhexanoic acid;

(R)-2-{[(5-indanyl)methyl]amino}-2,5,5-trimethylhexanoic acid;

(S)-2-{[(5-indanyl)methyl]amino}-2,5,5-trimethylhexanoic acid;

(S)-2-{[(4-aza-6-indanyl)methyl]amino}-2,5,5-trimethylhexanoic acid;

(S)-2-{[(2,3-dihydro-1,4-benzodioxin-6-yl)methyl]amino}-2,5,5-trimethylhexanoic acid;

(S)-2-{[(6-chromanyl)methyl]amino}-2,5,5-trimethylhexanoic acid;

(S)-2-{[(6-isochromanyl)methyl]amino}-2,5,5-trimethylhexanoic acid;

(S)-2-{[(7-isochromanyl)methyl]amino}-2,5,5-trimethylhexanoic acid;

(S)-2-{[(7-chromanyl)methyl]amino}-2,5,5-trimethylhexanoic acid;

or a pharmaceutically acceptable salt, solvate, hydrate, tautomer, optical isomer, N-oxide, and/or prodrug thereof.

According to a second aspect of the invention, there is a pharmaceutical composition comprising a compound according to the invention and a pharmaceutically acceptable carrier, excipient, and/or diluent.

According to a third aspect of the invention, there is provided a compound or pharmaceutical composition according to the invention for use in therapy.

According to a fourth aspect of the invention, there is provided a compound or pharmaceutical composition according to the invention for use in the treatment or prevention of a neurodegenerative disorder, a psychiatric disorder, an inflammatory disorder, a lysosomal storage disorder, a cancer, pain, diabetes mellitus, retinopathies such as diabetic retinopathy, brain tumours, glaucoma, uveitis, cardiovascular diseases, kidney disease, psoriasis, hereditary eye conditions, chronic pain, hearing loss or diseases characterized by misfolded tau.

Preferably, the neurodegenerative disorder is selected from motor neuron diseases, Frontotemporal Lobar Degeneration (FTLD), frontotemporal dementia, Alzheimer's disease, Parkinson's disease, Huntington's disease, prion diseases such as Creutzfeldt-Jakob disease (CJD), acute brain injury, spinal cord injury and stroke, preferably wherein the motor neuron disease is selected from amyotrophic lateral sclerosis (ALS), Primary Lateral Sclerosis, and Progressive Muscular Atrophy.

The neurodegenerative disorder is preferably a neurodegenerative disorder characterised by misfolded TAR DNA binding protein 43 (tdp-43). In other words, the neurodegenerative disease is characterized by truncated tdp-43 and inclusion bodies. Examples of such diseases include amyotrophic lateral sclerosis, Alzheimer's disease, Frontotemporal Lobar Degeneration, and frontotemporal dementia.

Preferably, the psychiatric disorder is selected from bipolar disorder, major depression, post-traumatic stress disorder, and anxiety disorders;

Preferably, the inflammatory disorder may be selected from inflammatory diseases and neuroinflammation.

Preferably, the lysosomal storage disorder is selected from the group consisting of NCL/Batten Disease caused by mutations in CLN gene *CLN1 (PPT1), CLN2 (TPP1), CLN3, CLN4 (DNAJC5), CLN5, CLN6, CLN7 (MFSD8), CLN8, CLN10 (CTSD), CLN11, CLN12 (ATP13A2), CLN13 (CTSF), CLN14 (KCTD7), CLCN6,* and/or *SGSH*; Pompe disease, Fabry disease, Gaucher disease, Niemann-Pick disease Types A, B, and C; GM1 gangliosidosis, GM2 gangliosidosis (including Sandhoff and Tay-Sachs), mucopolysachariddoses (MPS) types I (Hurler disease)/II (Hunter disease)/IIIa (Sanfilippo A)/IIIB (Sanfilippo B)/IIIc (Sanfilippo C)/IIId (Sanfilippo D)/IVA (Morqui" A)/'VB/VI/VII (Sly)/IX, mucolipisosis III (I-cell) and IV, multiple sulfatase deficiency; sialidosis, galactosialidosis, α-mannosidosis, β-mannosidosis, apartylglucosaminuria, fucosidosis, Schindler disease, metachromatic leukodystrophy caused by deficiencies in either arylsulfatase A or Saposin B, globoid cell leukodystrophy (Krabbe disease), Farber lipogranulomatosis, Wolman and cholesteryl ester storage disease, pycnodystostosis, cystinosis, Salla disease, Danon disease, Griscelli disease Types ½/3, Hermansky Pudliak Disease, and Chédiak-Higashi syndrome.

Preferably, the cancer is selected from breast cancer, lung cancer, ovarian cancer, prostate cancer, thyroid cancer, pancreatic cancer, glioblastoma and colorectal cancer.

Preferably, the cardiovascular disease is selected from atherosclerosis, cardiomyopathy, heart attack, arrhythmias, heart failure, and ischemic heart disease.

Preferably, the hearing loss is selected from noise-induced hearing loss, ototoxicity induced hearing loss, age-induced hearing loss, idiopathic hearing loss, tinnitus and sudden hearing loss.

According to a fifth aspect of the invention, there is provided the use of the compound according to the invention for the manufacture of a medicament for the treatment or prevention of a neurodegenerative disorder, a psychiatric disorder, an inflammatory disorder, a lysosomal storage disorder, a cancer, pain, diabetes mellitus, retinopathies such as diabetic retinopathy, brain tumours, glaucoma, uveitis, cardiovascular diseases, kidney disease, psoriasis, hereditary eye conditions, chronic pain, hearing loss or diseases characterized by misfolded tau.

According to a sixth aspect of the invention, there is provided a method for the treatment or prevention of a disease or condition responsive to sortilin modulation comprising administering a therapeutically effective amount of a compound or pharmaceutical composition according to the invention.

The compounds of the invention may include isotopically-labelled and/or isotopically-enriched forms of the

compounds. The compounds of the invention herein may contain unnatural proportions of atomic isotopes at one or more of the atoms that constitute such compounds. Examples of isotopes that can be incorporated into the disclosed compounds include isotopes of hydrogen, carbon, nitrogen, oxygen, phosphorus, sulfur, chlorine, such as $^{2}H$, $^{3}H$, $^{11}C$, $^{13}C$, $^{14}C$, $^{13}N$, $^{15}O$, $^{17}O$, $^{32}P$, $^{35}S$, $^{18}F$, $^{36}Cl$.

The compounds of the invention may be used as such or, where appropriate, as pharmacologically acceptable salts (acid or base addition salts) thereof. The pharmacologically acceptable addition salts mentioned below are meant to comprise the therapeutically active non-toxic acid and base addition salt forms that the compounds are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids such as formic acid, acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, p-aminosalicylic acid, pamoic acid, benzoic acid, ascorbic acid and the like. Compounds that have acidic properties can be converted to their pharmaceutically acceptable basic addition salts by treating the acid form with an appropriate base. Exemplary base addition salt forms are the sodium, potassium, calcium salts, and salts with pharmaceutically acceptable amines such as, for example, ammonia, alkylamines, benzathine, and amino acids, such as, e.g. arginine and lysine. The term addition salt as used herein also comprises solvates which the compounds and salts thereof are able to form, such as, for example, hydrates, alcoholates and the like.

Throughout the present disclosure, a given chemical formula or name shall also encompass all pharmaceutically acceptable salts, solvates, hydrates, N-oxides, and/or prodrug forms thereof. It is to be understood that the compounds of the invention include any and all hydrates and/or solvates of the compound formulas. It is appreciated that certain functional groups, such as the hydroxy, amino, and like groups form complexes and/or coordination compounds with water and/or various solvents, in the various physical forms of the compounds. Accordingly, the above formulas are to be understood to include and represent those various hydrates and/or solvates.

Compounds of the invention also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, amide - imidic acid pairs, enamine - imine pairs, and annular forms where a proton can occupy two or more positions of a heterocyclic system, for example, 1H- and 3H-imidazole, 1H, 2Hand 4H- 1,2,4-triazole, 1H- and 2H- isoindole, and 1H- and 2H-pyrazole. Tautomeric forms can be in equilibrium or sterically locked into one form by appropriate substitution.

The compounds described herein can be asymmetric (e.g. having one or more stereogenic centres). All stereoisomers, such as enantiomers and diastereomers, are intended unless otherwise indicated. Compounds of the present invention that contain asymmetrically substituted carbon atoms can be isolated in optically active or racemic forms. Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present invention. Cis- and trans-geometric isomers of the compounds of the present invention are described and may be isolated as a mixture of isomers or as separated isomeric forms.

In the case of the compounds which contain an asymmetric carbon atom, the invention relates to the D form, the L form, and D,L mixtures and also, where more than one asymmetric carbon atom is present, to the diastereomeric forms. Those compounds of the invention which contain asymmetric carbon atoms, and which as a rule accrue as racemates, can be separated into the optically active isomers in a known manner, for example using an optically active acid. However, it is also possible to use an optically active starting substance from the outset, with a corresponding optically active or diastereomeric compound then being obtained as the end product.

Preferably, the compounds of Formula (I) are compounds of Formula (Ia):

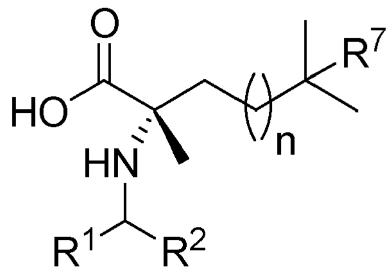

(Ia)

The term "prodrugs" refers to compounds that may be converted under physiological conditions or by solvolysis to a biologically active compound of the invention. A prodrug may be inactive when administered to a subject in need thereof, but is converted in vivo to an active compound of the invention. Prodrugs are typically rapidly transformed in vivo to yield the parent compound of the invention, e.g. by hydrolysis in the blood. The prodrug compound usually offers advantages of solubility, tissue compatibility or delayed release in a mammalian organism (see Silverman, R. B., The Organic Chemistry of Drug Design and Drug Action, 2nd Ed., Elsevier Academic Press (2004), page 498 to 549). Prodrugs of a compound of the invention may be prepared by modifying functional groups, such as a hydroxy, amino or mercapto groups, present in a compound of the invention in such a way that the modifications are cleaved, either in routine manipulation or in vivo, to the parent compound of the invention. Examples of prodrugs include, but are not limited to, acetate, formate and succinate derivatives of hydroxy functional groups or phenyl carbamate derivatives of amino functional groups.

The compounds of the invention may be sortilin inhibitors, binders, modulators or antagonists. As used herein, the term "sortilin antagonist", "sortilin inhibitor", "sortilin binder" or "sortilin modulator" (used interchangeably) refers to a substance that interferes with, blocks, or otherwise attenuates the effect of, a sortilin protein binding to progranulin, or neurotensin or another extracellular ligand, or a pro-neurotrophin (e.g., pro-NGF, proNT3, pro-BDNF) or preventing the formation of the trimeric complex between sortilin, p75NTR and the pro-neurotrophin. The term "sortilin antagonist" also includes a substance or agent that interferes with the formation of a high affinity trimeric complex. In the latter scenario, it is recognised that a trimeric complex may be formed in that sortilin can bind to p75NTR (but not pro-NGF) and p75NTR can simultaneously bind the NGF domain of pro-NGF. However, the resulting trimeric complex may be of lower affinity for its receptor and as a result have significantly reduced capacity to stimulate apoptosis via the mechanism described above. Skeldal et al. (2012) demonstrated that the apoptotic function of the trimeric complex is abolished when sortilin is devoid in its intracellular domain. The term "sortilin antagonist" also includes a substance or agent that interferes with, blocks, or otherwise attenuates the effect of, a sortilin protein interacting with p75NTR. This interaction may be completely prevented, in which case the trimeric complex is prevented from forming, or only partially prevented, in which case the trimeric complex may be formed but may have reduced biological potency. Skeldal *et al* showed that complex formation between sortilin and p75NTR relies on contact points in the extracellular domains of the receptors and that the interaction critically depends on an extracellular juxtamembrane 23-amino acid sequence of p75NTR. Thus, the sortilin antagonist may interfere with this 23-amino acid sequence or proximal sequences in the molecules. "Sortilin antagonists" may act as ligand cellular uptake inhibitors wherein the ligands may be progranulin, neurotensin, BDNF etc.

The compounds of the invention may cross the blood brain barrier and therefore may be particularly useful in the treatment or prevention of diseases of the central nervous system, including neurodegenerative disorders selected from motor neuron diseases, Frontotemporal Lobar Degeneration (FTLD), frontotemporal dementia, Alzheimer's disease, Parkinson's disease, Huntington's disease, prion diseases such as Creutzfeldt-Jakob disease (CJD), acute brain injury, spinal cord injury and stroke; a psychiatric disorder selected from bipolar disorder, major depression, post-traumatic stress disorder, and anxiety disorders; hearing loss selected from noise-induced hearing loss, ototoxicity induced hearing loss, age-induced hearing loss, idiopathic hearing loss, tinnitus and sudden hearing loss; brain tumours, retinopathies, glaucoma, neuroinflammation, chronic pain and diseases characterized by misfolded tau.

The compounds of the invention may have a $K_{puu}$ of more than 0.1, such as between 0.1 and 10, between 0.1 and 5, between 0.1 and 3, between 0.1 and 2, between 0.1 and 1, between 0.1 and 0.8, between 0.1 and 0.6, between 0.1 and 0.5, between 0.1 and 0.4, between 0.1 and 0.3, or between 0.1 and 0.2.

The term "treatment" as used herein may include prophylaxis of the named disorder or condition, or amelioration or elimination of the disorder once it has been established. The term "prevention" refers to prophylaxis of the named disorder or condition.

Methods delineated herein include those wherein the subject is identified as in need of a particular stated treatment. Identifying a subject in need of such treatment can be in the judgment of a subject or a health care professional

and can be subjective (e.g. opinion) or objective (e.g. measurable by a test or diagnostic method).

In other aspects, the methods herein include those further comprising monitoring subject response to the treatment administrations. Such monitoring may include periodic imaging or sampling of subject tissue, fluids, specimens, cells, proteins, chemical markers, genetic materials, etc. as markers or indicators of the treatment regimen. In other methods, the subject is pre-screened or identified as in need of such treatment by assessment for a relevant marker or indicator of suitability for such treatment.

The invention provides a method of monitoring treatment progress. The method includes the step of determining a level of diagnostic marker (Marker) (e.g. any target or cell type delineated herein modulated by a compound herein) or diagnostic measurement (e.g., screen, assay) in a subject suffering from or susceptible to a disorder or symptoms thereof delineated herein, in which the subject has been administered a therapeutic amount of a compound herein sufficient to treat the disease or symptoms thereof. The level of Marker determined in the method can be compared to known levels of Marker in either healthy normal controls or in other afflicted patients to establish the subject's disease status. In preferred embodiments, a second level of Marker in the subject is determined at a time point later than the determination of the first level, and the two levels are compared to monitor the course of disease or the efficacy of the therapy. In certain preferred embodiments, a pre-treatment level of Marker in the subject is determined prior to beginning treatment according to this invention; this pre-treatment level of Marker can then be compared to the level of Marker in the subject after the treatment commences, to determine the efficacy of the treatment.

A level of Marker or Marker activity in a subject may be determined at least once. Comparison of Marker levels, e.g., to another measurement of Marker level obtained previously or subsequently from the same patient, another patient, or a normal subject, may be useful in determining whether therapy according to the invention is having the desired effect, and thereby permitting adjustment of dosage levels as appropriate. Determination of Marker levels may be performed using any suitable sampling/expression assay method known in the art or described herein. Preferably, a tissue or fluid sample is first removed from a subject. Examples of suitable samples include blood, urine, tissue, mouth or cheek cells, and hair samples containing roots. Other suitable samples would be known to the person skilled in the art. Determination of protein levels and/or mRNA levels (e.g., Marker levels) in the sample can be performed using any suitable technique known in the art, including, but not limited to, enzyme immunoassay, ELISA, radiolabelling/assay techniques, blotting/chemiluminescence methods, real-time PCR, and the like.

For clinical use, the compounds disclosed herein are formulated into pharmaceutical compositions (or formulations) for various modes of administration. It will be appreciated that compounds of the invention may be administered together with a physiologically acceptable carrier, excipient, and/or diluent (i.e. one, two, or all three of these). The pharmaceutical compositions disclosed herein may be administered by any suitable route, preferably by oral, rectal, nasal, topical (including ophthalmic, buccal and sublingual), sublingual, transdermal, intrathecal, transmucosal or parenteral (including subcutaneous, intramuscular, intravenous and intradermal) administration. Other formulations may conveniently be presented in unit dosage form, e.g., tablets and sustained release capsules, and in liposomes, and may be prepared by any methods well known in the art of pharmacy. Pharmaceutical formulations are usually prepared by mixing the active substance, or a pharmaceutically acceptable salt thereof, with conventional pharmaceutically acceptable carriers, diluents or excipients. Examples of excipients are water, gelatin, gum arabicum, lactose, microcrystalline cellulose, starch, sodium starch glycolate, calcium hydrogen phosphate, magnesium stearate, talcum, colloidal silicon dioxide, and the like. Such formulations may also contain other pharmacologically active agents, and conventional additives, such as stabilizers, wetting agents, emulsifiers, flavouring agents, buffers, and the like. Usually, the amount of active compounds is between 0.1-95% by weight of the preparation, preferably between 0.2-20% by weight in preparations for parenteral use and more preferably between 1-50% by weight in preparations for oral administration. The formulations can be further prepared by known methods such as granulation, compression, microencapsulation, spray coating, Etc. The formulations may be prepared by conventional methods in the dosage form of tablets, capsules, granules, powders, syrups, suspensions, suppositories or injections. Liquid formulations may be prepared by dissolving or suspending the active substance in water or other suitable vehicles. Tablets and granules may be coated in a conventional manner. To maintain therapeutically effective plasma concentrations for extended periods of time, compounds disclosed herein may be incorporated into slow-release formulations.

The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

**DEFINITIONS**

As used herein, the term "sortilin" may refer to full length sortilin (also referred to as immature sortilin), comprising a signal peptide, a propeptide, a Vps10p domain, a 10CC domain, a transmembrane domain and a large cytoplasmic tail, having an amino acid sequence according to SEQ ID NO: 1 or SEQ ID NO: 2, or it may refer to mature sortilin, comprising a Vps10p domain, a 10CC domain, a transmembrane domain and a large cytoplasmic tail, having an amino acid sequence according to SEQ ID NO: 3, or a naturally occurring fragment, homologue or variant thereof. The term "sortilin" or "sortilin molecule" are used interchangeably herein. It is understood that sortilin is capable of interacting with a pro-neurotrophin molecule to form a sortilin/pro-neurotrophin complex. This sortilin/pro-neurotrophin complex may or may not be capable of interacting with a p75NTR molecule to form a trimeric complex comprising sortilin, pro-neurotrophin and p75NTR. It is understood that this trimeric complex may be responsible for adverse biological responses, such as stimulating apoptosis in retinal and ganglion cells, and controlling growth cone retraction of projecting axons (Jansen et al., 2007; Nykjaer et al., 2004; Santos et al., 2012; Skeldal et al., 2012).

As used herein, the term "pro-neurotrophin" refers to the larger precursors of neurotrophins, which undergo proteolytic cleavage to yield the mature form of the neurotrophin. Neurotrophins are a family of proteins that induce the survival, development and function of neurons, and are commonly referred to as growth factors. Pro-neurotrophins are biologically active and have distinct roles compared to their neurotrophin counterparts, such as induction of apoptosis. Examples of pro-neurotrophins include pro-NGF, pro-BDNF, proNT3 and proNT4. Pro-neurotrophins may also control synaptic plasticity. Whereas mature neurotrophins induce synaptic strength, in their proforms they may weaken synapses.

"Optional" or "optionally" means that the subsequently described event or circumstance may, but need not, occur, and that the description includes instances where the event or circumstance occurs and instances in which it does not.

The term "heteroatom" means O, N, or S.

The term "$(C_1-C_n)$alkyl" denotes a straight, branched, cyclic, or partially cyclic alkyl group having from 1 to n carbon atoms, i.e. 1, 2, 3... or n carbon atoms. For the "$(C_1-C_n)$alkyl" group to comprise a cyclic portion it should be formed of at least three carbon atoms. For parts of the range "$(C_1-C_n)$alkyl" all subgroups thereof are contemplated. For example, in the range $(C_1-C_4)$alkyl, all subgroups such as $(C_1-C_4)$alkyl, $(C_1-C_3)$alkyl, $(C_1-C_2)$alkyl, $(C_1)$alkyl, $(C_2-C_4)$alkyl, $(C_2-C_3)$alkyl, $(C_2)$alkyl, $(C_3-C_4)$alkyl, $(C_3)$alkyl, and $(C_4)$alkyl. Examples of "$C_1-C_4$ alkyl" include methyl, ethyl, n-propyl, isopropyl, cyclopropyl, n-butyl, isobutyl, sec-butyl, t-butyl, cyclobutyl etc.

The term "$(C_1-C_n)$ haloalkyl" denotes a $C_1-C_n$ alkyl as described above substituted with at least one halogen atom, which is preferably, F, Cl, Br and I, more preferably F and Cl, and most preferably F.

The term "$(C_1-C_n)$ hydroxyalkyl" denotes a $C_1-C_n$ alkyl as described above substituted with at least one -OH group.

The term "$(C_1-C_n)$alkoxy" denotes -O-($(C_1-C_n)$alkyl) in which a $(C_1-C_n)$alkyl group is as defined above and is attached to the remainder of the compound through an oxygen atom.

When a term denotes a range, for instance "1 to 4 carbon atoms" in the definition of $(C_1-C_4)$alkyl, each integer is considered to be disclosed, i.e. 1, 2, 3, and 4.

The term "halo" means a halogen atom, and is preferably, F, Cl, Br and I, more preferably F and Cl, and most preferably F.

"An effective amount" refers to an amount of a compound of the invention that confers a therapeutic effect on the treated subject. The therapeutic effect may be objective (i.e. measurable by some test or marker) or subjective (i.e. subject gives an indication of or feels an effect).

As used herein, the terms "administration" or "administering" mean a route of administration for a compound disclosed herein. Exemplary routes of administration include, but are not limited to, oral, intraocular, intravenous, intraperitoneal, intraarterial, and intramuscular. The preferred route of administration can vary depending on various factors, e.g. the components of the pharmaceutical composition comprising a compound disclosed herein, site of the potential or actual disease and severity of disease.

The terms "subject" and "patient" are used herein interchangeably. They refer to a human or another mammal (e.g., mouse, rat, rabbit, dog, cat, cattle, swine, sheep, horse or primate) that can be afflicted with or is susceptible to a disease or disorder but may or may not have the disease or disorder. It is preferred that the subject is human.

Compounds of the invention may be disclosed by the name or chemical structure. If a discrepancy exists between the name of a compound and its associated chemical structure, then the chemical structure prevails.

The invention will now be further illustrated by the following non-limiting examples. The specific examples below are to be construed as merely illustrative, and not limitative of the remainder of the disclosure in any way whatsoever. Without further elaboration, it is believed that one skilled in the art can, based on the description herein, utilise the present invention to its fullest extent. All references and publications cited herein are hereby incorporated by reference in their entirety.

## PREPARATION OF COMPOUNDS OF THE INVENTION

The compounds of the invention can be prepared according to the following General Synthetic Procedures scheme by methods well known and appreciated in the art. Suitable reaction conditions are well known in the art and appropriate substitutions of solvents and co-reagents are within the common general knowledge of the person skilled in the art. Likewise, it will be appreciated by those skilled in the art that synthetic intermediates may be isolated and/or purified by various well-known techniques as needed or desired, and that frequently, it will be possible to use various intermediates directly in subsequent synthetic steps with little or no purification. Furthermore, the skilled person will appreciate that in some circumstances, the orders in which moieties are introduced is not critical. The particular order of steps required to produce the compounds of formula (I) is dependent upon the particular compound being synthesized, the starting compound, and the relative liability of the substituted moieties as is well appreciated by those of ordinary skill in the art. All substituents, unless otherwise indicated, are as previously defined, and all reagents are well known and appreciated in the art.

Suitable starting materials, either optically active as single enantiomers or as racemic mixtures and protected amino acids of general formula AA-1 are either commercially available or may be prepared by a variety of methods. For example, as illustrated in the General Synthetic Procedure, **Scheme 1**, the carboxylic acid functionality of appropriately substituted amino acids of general formula AA-1, can be used as the free acid, PG = H, or protected as a suitable derivative, for example as a methyl ester. Functionalisation of the primary amine present in Int 1 can be done by a variety of methods, and for the purpose of exemplification, by a condensation step involving a suitably substituted carbonyl compound aldehyde or ketone to yield an imine type Int 2. When treated by a reductive reagent, as for example but not limited to, sodium triacetoxy borohydride in a suitable solvent mixture like acetic acid and dichloromethane the imine function can be reduced to afford an intermediate of type Int 3 that is a precursor of compounds of Formula (I) after liberation of the carboxylic acid functionality. Alternatively, an amino acid of type AA-2 can be used as starting material. By using, as previously discussed, a condensation step to obtain an intermediate of type Int 4, as shown in **Scheme 2,** that when treated with a suitable base, will lose its most acidic proton, resulting in a transient carbanion that can, thus, be quenched by an alkylating agent such as, but not limited to, Methyl iodide, resulting in a compound of type Int 2. When treated by a reductive reagent, as for example but not limited to, sodium triacetoxy borohydride in a suitable solvent mixture like acetic acid and dichloromethane the imine function can be reduced to afford an intermediate of type Int 3 or hydrolysed by treatment with an aqueous solution acidic or basic to provide the generic intermediate Int 5. As in **Scheme 1**, Compounds of Formula (I) can then be obtained directly from Int 3 by liberation of the acidic moiety, with or without, further functionalisation of the amine group.

Another possible approach from a readily amenable starting amino acid of the type AA-3, as represented in the general Synthetic Procedures **Scheme 3**, uses an alkylation step between the suitable protected AA-3 or Int 6 and a reagent of type, for exemplification purposes, 1-bromo-3,3-dimethylbutane to yield generic intermediates of type Int 2, previously discussed. Advantageously, intermediates of type AA-1 to AA-3 can be obtained by procedures known, to those skilled in the art, or purchased from commercial sources.

## GENERAL SYNTHETIC PROCEDURES

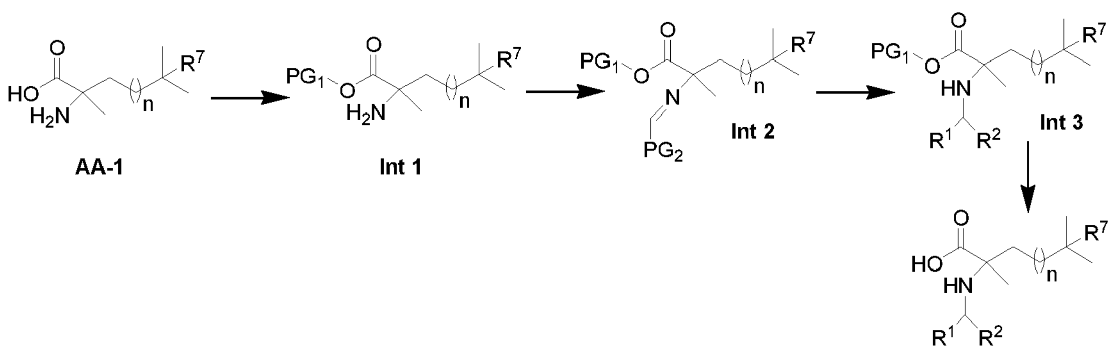

AA-1 Int 1 Int 2 Int 3 Formula (I)

**Scheme 1**

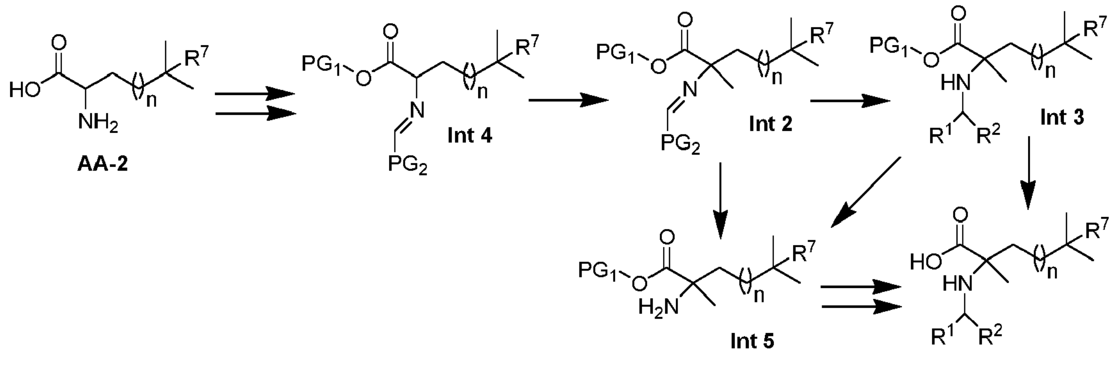

AA-2, Int 4, Int 2, Int 3, Int 5, Formula (I)

**Scheme 2**

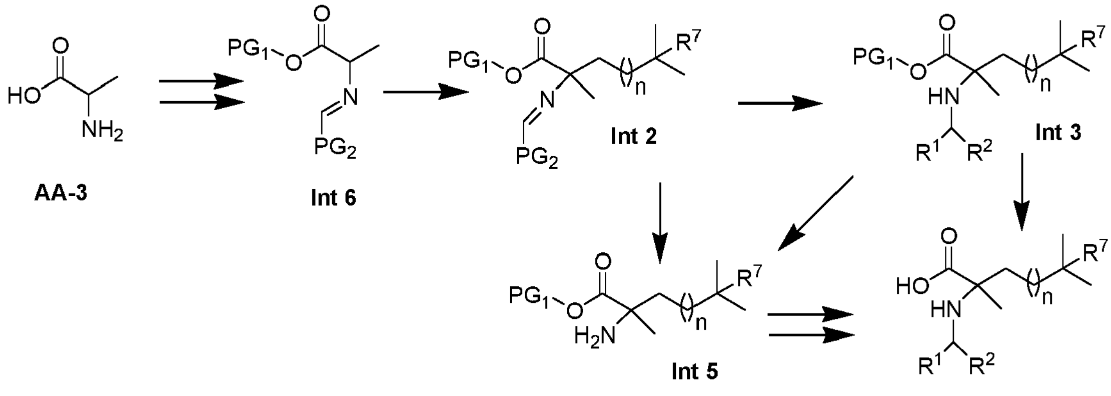

AA-3, Int 6, Int 2, Int 3, Int 5, Formula (I)

**Scheme 3**

The compounds of general formula (I) may be prepared by a variety of procedures, some of which are described below. The products of each step can then be recovered by conventional methods including extraction, evaporation, precipitation, chromatography, filtration, trituration, crystallisation and the like.

Compounds of general formula (I) may contain one or more stereogenic centres. Those can be introduced from available single enantiomers, optically active, starting materials of the type AA-1, AA-2, or AA-3. The integrity of the existing stereogenic centre can be confirmed by analytical techniques well known to those skilled in the art like for example chiral support high pressure chromatography. Alternatively, when racemic starting materials are used, it will be appreciated that if desired, single isomer products can be obtained as single enantiomers or as single diastereoisomers, by known techniques like preparative chiral support high pressure chromatography.

The skilled artisan will also appreciate that not all of the substituents in the compounds of formula (I) will tolerate certain reaction conditions employed to synthesise the compounds. These moieties may be introduced at a convenient point in the synthesis, or may be protected and then deprotected as necessary or desired, as is well known in the art. The skilled artisan will appreciate that the protecting groups may be removed at any convenient point in the synthesis of the compounds of the present invention. Methods for introducing or removing protecting groups used in this invention are well known in the art; see, for example, Greene and Wuts, Protective Groups in Organic Synthesis, 4th Ed., John Wiley and Sons, New York (2006).

**EXAMPLES**

**Abbreviations**

approx: approximately; aq: aqueous; br: broad; *ca.: circa;* CDI: 1,1'-Carbonyldiimidazole; CPME: cyclopentyl methyl ether; d: doublet; DCM: dichloromethane; DIC: *N*,*N*'-Di*i*sopropylcarbodiimide; dioxane: 1,4-dioxane; DIPEA: diisopropylethylamine; DMF: dimethylformamide; eq.: equivalent; $Et_3N$: triethylamine; EtOAc: ethyl acetate; EtOH: ethanol; Fmoc: fluorenylmethoxycarbonyl; Boc: *tert*-butoxycarbonyl; h: hours; min: minutes: HATU: 2-(3*H*-[1,2,3]triazolo[4,5-b]pyridin-3-yl)-1,1,3,3-tetramethyl isouronium hexafluorophosphate(V); HPLC: high performance liquid chromatography; IPA, isopropanol; LC: liquid chromatography; m: multiplet; M: molar, molecular ion; MeCN: acetonitrile; MeOH: methanol; MS: mass spectrometry; NMR: nuclear magnetic resonance; PDA: photodiode array; q: quartet; rt: room temperature (*ca.* 20 °C); $R_T$: retention time; s: singlet, solid; SPPS: solid phase peptide synthesis. t: triplet; TBAF: tetrabutylammonium fluoride; TBME: *tert*-butyl methyl ether; TFA: trifluoroacetic acid; THF: tetrahydrofuran; UPLC: ultra-performance liquid chromatography; UV: ultraviolet.

Other abbreviations are intended to convey their generally accepted meaning.

**General Experimental Conditions**

All starting materials and solvents were obtained either from commercial sources or prepared according to the literature citation. Reaction mixtures were magnetically stirred, and reactions performed at room temperature (*ca.* 20 °C) unless otherwise indicated.

Column chromatography was performed on an automated flash chromatography system, such as a CombiFlash Rf system, using pre-packed silica (40 μm) cartridges, unless otherwise indicated.

**Analytical Methods**

$^1$H-NMR spectra were recorded at 400 MHz on a Bruker Avance AV-I-400 or on a Broker Avance AV-II-400 instrument. Chemical shift values are expressed in ppm-values relative to tetramethylsilane unless noted otherwise. The following abbreviations or their combinations are used for multiplicity of NMR signals: br = broad, d = doublet, m = multiplet, q = quartet, quint = quintet, s = singlet and t = triplet.

Method 1: Apparatus: Waters IClass; Bin. Pump: UPIBSM, SM: UPISMFTN with SO; UPCMA, PDA: UPPDATC, 210-320 nm, SQD: ACQ-SQD2 ESI; ELSD: gaspressure 40 psi, drift tube temp: 50°C; column: Waters XSelect CSH C18, 50×2.1mm, 2.5μm, Temp: 25°C, Flow: 0.6 mL/min, Gradient: t0 = 5% B, t2.0min = 98% B, t2.7min = 98% B, Posttime: 0.3 min, EluentA: 10mM ammonium bicarbonate in water (pH=9.5), Eluent B: acetonitrile.

Method 2: Apparatus: Agilent 1260 Infinity, 1260 G1312B Bin. Pump, 1260 G1367E WPS, 1260 TCC G1316A Column Comp. 1260 G1315C DAD (210-320 nm, 210 and 220nm), PDA (210-320 nm), G6130B MSD ESI pos/neg (mass range 100-1000), Column: Waters XSelect CSH C18 (30×2.1mm 3.5μ), Flow: 1 ml/min; Column Temp: 25°C, Eluent A: 10 mM ammonium bicarbonate in water (pH 9), Eluent B: Acetonitrile, Gradient: t=0 min 5% B, t=1.6 min 98% B, t=3 min 98% B, Postrun: 1.3 min.

Method 3: Apparatus: Agilent Technologies G6130B Quadrupole; HPLC instrument type: Agilent Technologies 1290 preparative LC; Column: Waters XSelect CSH (C18, 100x30mm, 10μ); Flow: 55 mL/min; Column temp: RT; Eluent A: 0.1% formid acid in water; Eluent B: 100% acetonitrile lin. gradient: t=0 min 20% B, t=2 min 20% B, t=8.5 min 60% B, t=10 min 100% B, t=13 min 100% B; Detection: DAD (220-320 nm); Detection: MSD (ESI pos/neg) mass range: 100 - 1000; fraction collection based on MS and DAD.

Method 4: Apparatus: ACQ-SQD2; HPLC instrument type: Waters Modular Preparative HPLC System; column: Waters XSelect (C18, 100x30mm, 10μm); flow: 55 ml/min prep pump; column temp: RT; eluent A: 10mM ammonium bicarbonate in water pH=9.5, eluent B: 100% acetonitrile; detection: DAD (220-320 nm); detection: MSD (ESI pos/neg) mass range: 100 - 800; fraction collection based on MS and DAD.

Method 5: Apparatus: Waters IClass; Bin. Pump: UPIBSM, SM: UPISMFTN with SO; UPCMA, PDA: UPPDATC, 210-320 nm, SQD: MS: QDA ESI, pos/neg 100-800; column: Waters XSelect CSH C18, 50×2.1mm, 2.5μm, Temp: 25°C, Flow: 0.6 mL/min, Gradient: t0 = 5% B, t1.3min = 98% B, t1.7min = 98% B, Posttime: 0.3 min, Eluent A: 10mM ammonium bicarbonate in water (pH=9.5), Eluent B: acetonitrile. MS parameters: Source: ESI; Capillary: 2500 V; Cone: 20 V; Extractor: 3.0 V; RF: 2.5 V; Source Temp.: 150°C; Desolvation Temp.: 600°C; Cone Gas Flow: 80 L/Hr; Desolvation Gas Flow: 1000 L/Hr; Full MS scan: MS range 100-800 (positive and negative mode); scan: 0.4 sec.

Method 6: Apparatus: Waters I-Class UPLC, Binary Solvent Manager (BSM), Sample Manager-FTN (SM-FTN) and Sample Organizer (SO), Column Manager (CM-A), PDA 210-320nm, QDa ESI 100-800 (pos) 100-800 (neg), Column: XSelect CSH C18 XP (50×2.1mm 2.5μm) Flow: 0.6 ml/min; Column temp: 25°C, Eluent A: 10mM ammoniumbicarbonate in water (pH 9.5), Eluent B: acetonitrile, Gradient: t=0 min 5% B, t=2 min 98% B, t=2.7 min 98% B, Postrun: 0.3 min.

Method 7: Apparatus: Waters Acquity UPC2: Waters ACQ-ccBSM Binary Pump; Waters ACQ-CCM Convergence Manager; Waters ACQ-SM Sample Manager - Fixed Loop; Waters ACQ-CM Column Manager - 30S; Waters ACQ-PDA Photodiode Array Detector; Waters ACQ-ISM Make Up Pump, Waters Acquity QDa MS Detector; Column: Lux i-Cellulose-5 (100×4.6mm 5μm); Column temp: 35 °C; Flow: 2.5 ml/min; ABPR: 170 bar; Eluent A: CO2, Eluent B: Methanol + 20mM ammonia; Gradient: t=0 min 5% B, t=5 min 50% B, t=6 min 50% B; Detection: 210-320nm.

Method 8: Apparatus: Waters Prep 100 SFC UV/MS directed system; Waters 2998 Photodiode Array (PDA)

Detector; Waters Acquity QDa MS detector; Waters 2767. Sample Manager; Column: Phenomenex Lux i-Cellulos-5 (250x21.2 mm, 5 μm); Column temp: 35°C; Flow: 70 ml/min; ABPR: 120 bar; Eluent A: CO2, Eluent B: 20 mM Ammonia in Methanol; Detection: PDA (210-400 nm); Gradient: t=0 min 25% B; t=5 min 45% B. Sample concentration 20 mg/ml. Injection volume: 500 μl. Timed fraction collection.

Method 9: Apparatus: Waters Prep 100 SFC UV/MS directed system; Waters 2998 Photodiode Array (PDA) Detector; Waters Acquity QDa MS detector; Waters 2767 Sample Manager; Column: Diacel Chiralpak IC for SFC (250x20 mm, 5 μm); Column temp: 35°C; Flow: 70 ml/min; ABPR: 120 bar; Eluent A: CO2, Eluent B: 20 mM Ammonia in Methanol; Linear gradient: t=0 min 20% B, t=4.5 min 50% B; Detection: PDA (210-400 nm); Fraction collection: PDA TIC.

Method 10: Apparatus: Waters Acquity UPC2: Waters ACQ-ccBSM Binary Pump; Waters ACQ-CCM Convergence Manager; Waters ACQ-SM Sample Manager - Fixed Loop; Waters ACQ-CM Column Manager - 30S; Waters ACQ-PDA Photodiode Array Detector; Waters ACQ-ISM Make Up Pump, Waters Acquity QDa MS Detector; Column: Chiralpak IC (100×4.6mm 5μm); Column temp: 35°C; Flow: 2.5 ml/min; ABPR: 170 bar; Eluent A: CO2, Eluent B: Methanol + 20mM ammonia; Linear gradient: t=0 min 5% B, t=5 min 50% B, t=6 min 50% B; Detection: 210-320nm.

Method 11: Apparatus: Waters Acquity UPC2: Waters ACQ-ccBSM Binary Pump; Waters ACQ-CCM Convergence Manager; Waters ACQ-SM Sample Manager - Fixed Loop; Waters ACQ-CM Column Manager - 30S; Waters ACQ-PDA Photodiode Array Detector; Waters ACQ-ISM Make Up Pump, Waters Acquity QDa MS Detector; Column: phenomenex iAmylose-3 (100×4.6mm 5μm); Column temp: (35°C); Flow: (2.5 ml/min); ABPR: (170 bar); Eluent A: CO2, Eluent B: Methanol + 20mM ammonia; Linear gradient: t=0 min 5% B, t=5 min 50% B, t=6 min 50% B; Detection: 210-320nm.

Method 12: Apparatus: Waters Prep 100 SFC UV/MS directed system; Waters 2998 Photodiode Array (PDA) Detector; Waters Acquity QDa MS detector; Waters 2767 Sample Manager; Column: phenomenex iAmylose-3 (250×20 mm, 5 μm); Column temp: 35°C; Flow: 70 ml/min; ABPR: 170 bar; Eluent A: CO2, Eluent B: 20 mM Ammonia in Methanol; Linear gradient: t=0 min 20% B, t=4.5 min 50% B; Detection: PDA (210-400 nm); Fraction collection: PDA TIC.

## Examples 1, 2 and 3

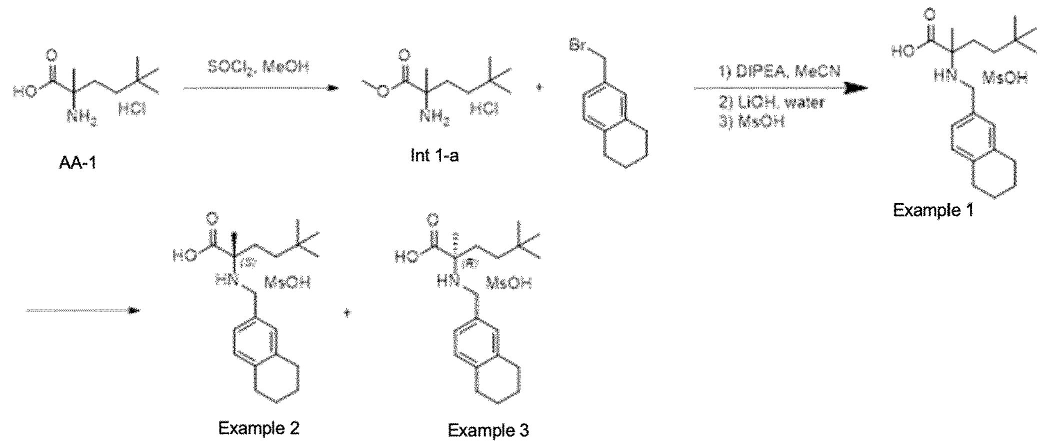

AA-1
Int 1-a
Example 1
Example 2
Example 3

### *Synthesis of methyl 2-amino-2,5,5-trimethylhexanoate hydrochloride (int-8)*

To an ice-cooled solution of 2-amino-2,5,5-trimethylhexanoic acid hydrochloride (250 mg, 1.192 mmol) in methanol (1 ml) under argon atmosphere was added dropwise thionyl chloride (0.261 ml, 3.58 mmol, 3.0 equiv.). The mixture was stirred at 0°C for 15 min and then at rt for 3h. A white thick suspension had formed. The suspension was diluted with little MeOH and stirred overnight. Thionyl chloride (0.261 ml, 3.58 mmol) was added additionally and after addition the mixture was heated under reflux for 4.5h. The mixture was allowed to cool to rt and solvent evaporated, MeCN added and solvent evaporated, affording methyl 2-amino-2,5,5-trimethylhexanoate hydrochloride (233 mg, 1.041 mmol, 87% yield) as an off-white turbid oil. LCMS (Method 2, 1.782min; M+H-HCl = 188.1; calcd. 188.2). $^1$H-NMR (400 MHz, DMSO) δ 8.53 (s, 2H), 3.77 (s, 3H), 1.83 - 1.70 (m, 2H), 1.47 (s, 3H), 1.33 - 1.20 (m, 1H), 1.06 - 0.93 (m, 1H), 0.85 (s, 9H).

***Synthesis of 2,5,5-trimethyl-2-(((5,6,7,8-tetrahydronaphthalen-2-yl)methyl)amino)hexanoic acid compound with methanesulfonic acid (Example 1)***

6-(bromomethyl)-1,2,3,4-tetrahydronaphthalene (131 mg, 0.581 mmol) and DIPEA (304 μl, 1.743 mmol, 3 Eq) were added to a suspension of methyl 2-amino-2,5,5-trimethylhexanoate hydrochloride (130 mg, 0.581 mmol) in MeCN (1.5 mL). Mixture heated at 80°C for 3h. The mixture was allowed to cool to rt, diluted with water (1.5 mL) and lithium hydroxide monohydrate (122 mg, 2.91 mmol, 7.5 Eq) were added. The mixture was heated at 80°C for 2d. The crude was filtered and purified by basic preparative prep (Method 4). Purified fractions were concentrated in the Genevac, and reformatted, redissolved in MeCN after weighing (0.05M) and a 0.1M MsOH in MeCN (1 equiv.) solution was added. Little water was added and solvent evaporated, affording 2,5,5-trimethyl-2-(((5,6,7,8-tetrahydronaphthalen-2-yl)methyl)amino)hexanoic acid compound with methanesulfonic acid (6.3 mg, 0.015 mmol, 2.62 % yield) as a white solid. LCMS Method 1, 1.247 min; M+H-MsOH= 318.5, calcd. 318.2. $^1$H NMR (400 MHz, DMSO) δ 9.00 (s, 2H), 7.18 (d, *J* = 6.3 Hz, 2H), 7.12 (d, *J* = 8.4 Hz, 1H), 4.14 - 3.88 (m, 2H), 2.78 - 2.69 (m, 4H), 2.29 (s, 3H, MsOH), 1.88 (td, *J* = 13.1, 4.6 Hz, 1H), 1.83 - 1.77 (m, 1H), 1.77 - 1.70 (m, 4H), 1.53 (s, 3H), 1.25 (dt, *J* = 12.9, 6.4 Hz, 1H), 1.17 (td, *J* = 13.3, 4.5 Hz, 1H), 0.87 (s, 9H).

***Synthesis of (S)-2,5,5-trimethyl-2-(((5,6,7,8-tetrahydronaphthalen-2-yl)methyl)amino)hexanoic acid compound with methanesulfonic acid (Example 2) and (R)-2,5,5-trimethyl-2-(((5,6,7,8-tetrahydronaphthalen-2-yl)methyl)amino)hexanoic acid with methanesulfonic acid (Example 3)***

methyl 2,5,5-trimethyl-2-(((5,6,7,8-tetrahydronaphthalen-2-yl)methyl)amino) hexanoate (516 mg, 1.557 mmol) was purified by SFC (method 8). Purified fractions were combined, evaporated, redissolved in MeCN after weighing (0.05M) and a 0.1M MsOH in MeCN (1 equiv.) solution was added. Little water was added and freeze-dried, affording (*S*)-2,5,5-trimethyl-2-(((5,6,7,8-tetrahydronaphthalen-2-yl)methyl)amino)hexanoic acid compound with methanesulfonic acid (40.7 mg, 0.098 mmol, 6.32 % yield, >95% EE). LCMS (Method 6, 1.291 min; M+H- MsOH= 318.3, calcd. 318.2). LCMS (Method 7, 4.080 min; M+H- MsOH= 318.1; calcd. 318.2). $^1$H-NMR (400 MHz, DMSO) δ 14.69 - 13.65 (m, 1H), 9.03 (s, 2H), 7.24 - 7.16 (m, 2H), 7.13 (d, J = 8.4 Hz, 1H), 4.18 - 3.88 (m, 2H), 2.79 - 2.68 (m, 4H), 2.30 (s, 3H, MsOH), 1.90 (td, J = 13.3, 4.6 Hz, 1H), 1.85 - 1.77 (m, 1H), 1.77 - 1.72 (m, 4H), 1.54 (s, 3H), 1.26 (td, J = 13.3, 4.0 Hz, 1H), 1.16 (td, J = 12.9, 4.6 Hz, 1H), 0.87 (s, 9H).

And (R)-2,5,5-trimethyl-2-(((5,6,7,8-tetrahydronaphthalen-2-yl)methyl)amino) hexanoic acid compound with methanesulfonic acid (36.6 mg, 0.088 mmol, 5.69 % yield, >95% EE). LCMS (Method 6, 1.293 min; M+H- MsOH= 318.2, calcd. 318.2). LCMS (Method 7, 4.846 min; M+H- MsOH= 318.1; calcd. 318.2). $^1$H-NMR (400 MHz, DMSO) δ 9.69 - 8.24 (m, 2H), 7.21 - 7.15 (m, 2H), 7.11 (d, 1H), 4.05 (d, J = 12.6 Hz, 1H), 3.92 (d, J = 12.5 Hz, 1H), 2.73 (s, 4H), 2.29 (s, 3H, MsOH), 1.87 (td, J = 12.5, 3.9 Hz, 1H), 1.81 - 1.76 (m, 1H), 1.76 - 1.69 (m, 4H), 1.50 (s, 3H), 1.26 (td, J = 13.0, 4.6 Hz, 1H), 1.17 (dt, J = 12.9, 6.5 Hz, 1H), 0.87 (s, 9H).

| Ex. | Structure | Yield | LCMS | NMR |
|---|---|---|---|---|
| **1** | 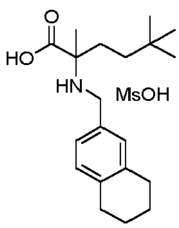 | 6.3 mg (2% yield, 97.54% purity) | Method 1, 1.247 min; M+H-MsOH= 318.5, calcd. 318.2 | $^1$H NMR (400 MHz, DMSO) δ 9.00 (s, 2H), 7.18 (d, *J* = 6.3 Hz, 2H), 7.12 (d, *J* = 8.4 Hz, 1H), 4.14 - 3.88 (m, 2H), 2.78 - 2.69 (m, 4H), 2.29 (s, 3H, MsOH), 1.88 (td, *J* = 13.1, 4.6 Hz, 1H), 1.83 - 1.77 (m, 1H), 1.77 - 1.70 (m, 4H), 1.53 (s, 3H), 1.25 (dt, *J* = 12.9, 6.4 Hz, 1H), 1.17 (td, *J* = 13.3, 4.5 Hz, 1H), 0.87 (s, 9H). |
| **2** | 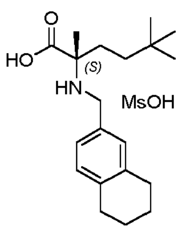 | 40.7 mg, 6% yield, 97.21% purity | Method 6, 1.291 min; M+H-MsOH= 318.3, calcd. 318.2 Method 7, 4.080 min; M+H-MsOH= 318.1; calcd. 318.2 | $^1$H-NMR (400 MHz, DMSO) δ 14.69-13.65 (m, 1H), 9.03 (s, 2H), 7.24 - 7.16 (m, 2H), 7.13 (d, *J* = 8.4 Hz, 1H), 4.18 - 3.88 (m, 2H), 2.79 - 2.68 (m, 4H), 2.30 (s, 3H, MsOH), 1.90 (td, *J* = 13.3, 4.6 Hz, 1H), 1.85 - 1.77 (m, 1H), 1.77 - 1.72 (m, 4H), 1.54 (s, 3H), 1.26 (td, *J* = 13.3, 4.0 Hz, 1H), 1.16 (td, *J* = 12.9, 4.6 Hz, 1H), 0.87 (s, 9H). |

(continued)

| Ex. | Structure | Yield | LCMS | NMR |
|---|---|---|---|---|
| **3** | 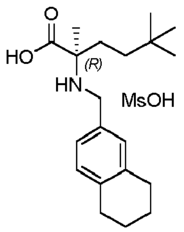 | 36.6 mg, 5% yield, 97.54% purity | Method 6, 1.293 min; M+H-MsOH= 318.2, calcd. 318.2 Method 7, 4.846 min; M+H-MsOH= 318.1; calcd. 318.2 | $^1$H NMR (400 MHz, DMSO) δ 9.69 - 8.24 (m, 2H), 7.21 - 7.15 (m, 2H), 7.11 (d, 1H), 4.05 (d, *J* = 12.6 Hz, 1H), 3.92 (d, *J* = 12.5 Hz, 1H), 2.73 (s, 4H), 2.29 (s, 3H, MsOH), 1.87 (td, *J* = 12.5, 3.9 Hz, 1H), 1.81 - 1.76 (m, 1H), 1.76 - 1.69 (m, 4H), 1.50 (s, 3H), 1.26 (td, *J* = 13.0, 4.6 Hz, 1H), 1.17 (dt, *J* = 12.9, 6.5 Hz, 1H), 0.87 (s, 9H). |

The following **Examples 4 to 8** were prepared in an analogous manner to example 1, starting from the corresponding bromide. The compounds were prepared as racemic mixtures.

| Ex. | Structure | Yield | LCMS | NMR |
|---|---|---|---|---|
| **4** | 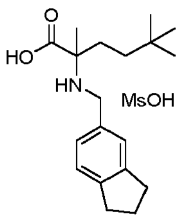 | 13.1 mg (9% yield, 99.04% purity) | Method 1, 1.168 min; M+H-MsOH= 304.5; calcd. 304.2 | $^1$H-NMR (400 MHz, DMSO) δ 7.31 (s, 1H), 7.24 (d, *J* = 7.6 Hz, 1H), 7.18 (d, *J* = 7.5 Hz, 1H), 3.95 - 3.78 (m, 2H), 2.91 - 2.78 (m, 4H), 2.29 (s, 3H, MsOH), 2.02 (p, *J* = 7.3 Hz, 2H), 1.79 - 1.69 (m, 1H), 1.69 - 1.57 (m, 1H), 1.36 (s, 3H), 1.30 - 1.08 (m, 2H), 0.85 (s, 9H). |
| **5** | 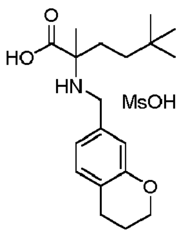 | 34.5 mg (13% yield, 99.78% purity | Method 1, 1.088 min; M+H-MsOH= 320.3; calcd. 320.2 | $^1$H-NMR (400 MHz, DMSO) δ 9.82 - 8.26 (m, 1H), 7.10 (d, *J* = 7.7 Hz, 1H), 6.91 (dd, *J* = 7.7, 1.8 Hz, 1H), 6.87 (d, *J* = 1.7 Hz, 1H), 4.14 (dd, *J* = 5.8, 4.4 Hz, 2H), 4.01 (d, *J* = 12.6 Hz, 1H), 3.89 (d, *J* = 12.6 Hz, 1H), 2.74 (t, *J* = 6.4 Hz, 2H), 2.29 (s, 3H, MsOH), 1.94 - 1.87 (m, 2H), 1.89 - 1.79 (m, 1H), 1.74 (td, *J* = 13.2, 4.7 Hz, 1H), 1.47 (s, 3H), 1.25 (td, *J* = 13.0, 4.7 Hz, 1H), 1.16 (td, *J* = 12.9, 4.7 Hz, 1H), 0.86 (s, 9H). |
| **6** | 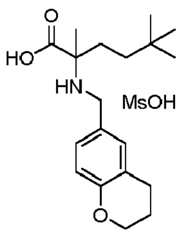 | 71.8 mg (38% yield, 99.89% purity | Method 6, 1.060 min; M+H-MsOH= 320.2; calcd. 320.2 | $^1$H-NMR (400 MHz, DMSO) δ 8.99 (s, 2H), 7.19 - 7.15 (m, 2H), 6.79 (d, *J* = 8.9 Hz, 1H), 4.15 (dd, *J* = 5.9, 4.3 Hz, 2H), 4.10 - 4.00 (m, 1H), 3.97 - 3.89 (m, 1H), 2.75 (t, *J* = 6.5 Hz, 2H), 2.32 (s, 3H, MsOH), 1.97 - 1.85 (m, 3H), 1.83 - 1.72 (m, 1H), 1.53 (s, 3H), 1.25 (td, *J* = 13.0, 6.5 Hz, 1H), 1.16 (td, *J* = 12.9, 4.7 Hz, 1H), 0.87 (s, 9H). |
| **7** | 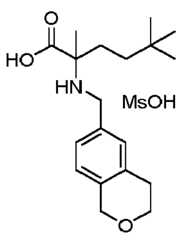 | 40.6 mg (39% yield, 99.85% purity | Method 6, 0.960 min; M+H-MsOH= 320.2; calcd. 320.2 | $^1$H-NMR (400 MHz, DMSO) δ 9.07 (s, 2H), 7.28 (dd, *J* = 7.8, 1.8 Hz, 1H), 7.22 (d, *J* = 7.8 Hz, 1H), 7.16 (d, *J* = 1.8 Hz, 1H), 4.70 (s, 2H), 4.17 - 4.06 (m, 1H), 4.05 - 3.95 (m, 1H), 3.89 (t, *J* = 5.7 Hz, 2H), 2.81 (t, *J* = 5.8 Hz, 2H), 2.31 (s, 3H, MsOH), 1.89 (dt, *J* = 13.3, 6.5 Hz, 1H), 1.79 (td, *J* = 13.3, 4.6 Hz, 1H), 1.54 (s, 3H), 1.26 (td, *J* = 12.9, 4.5 Hz, 1H), 1.16 (td, *J* = 12.9, 4.6 Hz, 1H), 0.87 (s, 9H). |
| **8** | 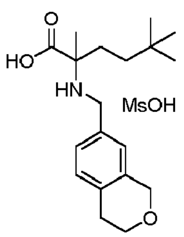 | 28.3 mg (44% yield, 99.65% purity | Method 6, 0.954 min; M+H-MsOH= 320.2; calcd. 320.2 | $^1$H-NMR (400 MHz, DMSO) δ 9.08 (s, 2H), 7.29 - 7.25 (m, 2H), 7.12 (d, *J* = 8.1 Hz, 1H), 4.70 (s, 2H), 4.17 - 4.07 (m, 1H), 4.04 - 3.94 (m, 1H), 3.89 (t, *J* = 5.7 Hz, 2H), 2.81 (t, *J* = 5.7 Hz, 2H), 2.32 (s, 3H, MsOH), 1.91 (td, *J* = 13.2, 4.7 Hz, 1H), 1.80 (td, *J* = 14.0, 5.2 Hz, 1H), 1.54 (s, 3H), 1.27 (td, *J* = 12.8, 4.5 Hz, 1H), 1.16 (td, *J* = 12.9, 4.7 Hz, 1H), 0.87 (s, 9H). |

The following **Examples 9 to 16** can be prepared in an analogous manner to the previous examples.

| Example | Structure |
|---|---|
| **9** | 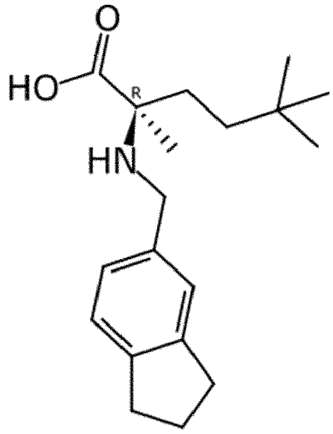 |
| **10** | 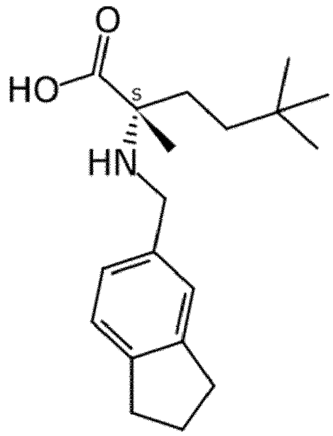 |
| **11** | 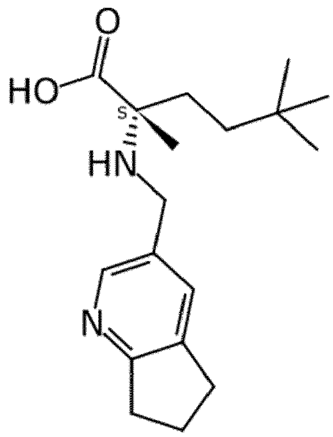 |

(continued)

| Example | Structure |
|---|---|
| 12 | 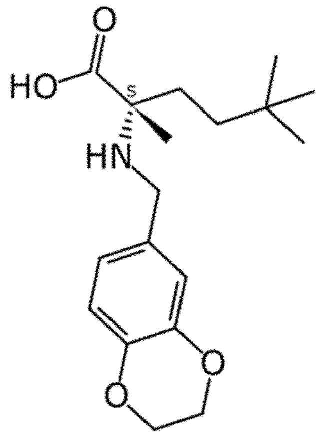 |
| 13 | 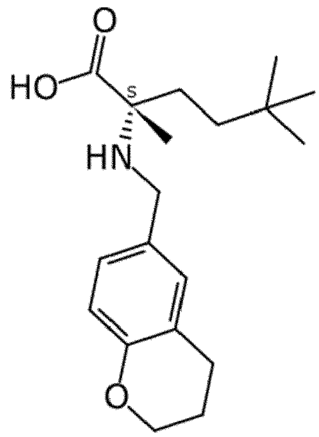 |
| 14 | 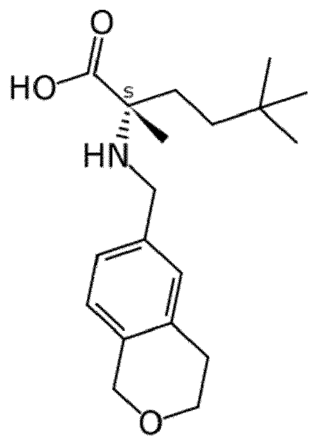 |

(continued)

| Example | Structure |
|---|---|
| **15** | 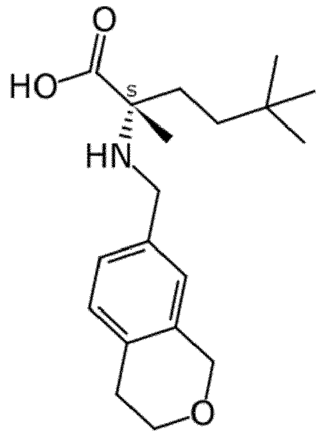 |
| **16** | 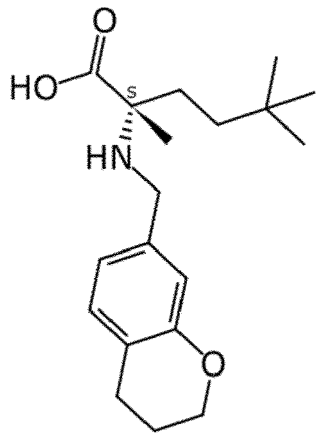 |

## BIOLOGICAL DATA

### Neurotensin scintillation proximity assay

The exemplified compounds of the invention were tested in a Neurotensin (NTS) scintillation proximity assay (SPA). The $IC_{50}$ data is shown in the table below. NTS, which is a 13 amino acid neuropeptide, is a sortilin ligand. The $IC_{50}$ is a measure of the amount of the compound required to inhibit the binding of NTS to sortilin by 50%. The skilled person will recognise that the lower the $IC_{50}$ value, the less of the compound needed to achieve the desired effect, and as a result, the chances of undesirable off-target effects are reduced.

Compound affinity was determined by measuring the displacement of [$^3$H]-neurotensin binding to h-Sortilin in SPA format. Total volume of 40 μl in 50 mM HEPES pH 7.4 assay buffer containing 100 mM NaCl, 2.0 mM CaCl2, 0.1% BSA and 0.1% Tween-20. Compound pre-incubation for 30 minutes at room temperature with 150 nM of 6his-Sortilin before 5 nM [3H]-Neurotensin and Ni chelate imaging beads (Perkin Elmer) were added, after 6 hours the plate was read on a ViewLux with 360 s exposure time. Dose-response evaluation of compounds was performed with 8 concentrations of drugs (covering 3 decades). $IC_{50}$ values were calculated by nonlinear regression using the sigmoid concentration-response (variable slope) using CDD Vault software. All values reported are average of at least 2 determinations.

### Human Sortilin GCI binding assay

The exemplified compounds of the invention were tested in a Grating-Coupled Interferometry (GCI) Sortilin binding assay. The GCI data is shown in Table 1 below. The Equilibrium dissociation constant ($K_D$) is a measure of the propensity of a complex (i.e: a ligand and receptor) to dissociate and is used to describe the affinity of an interaction between the constituents of the complex (i.e: a ligand and receptor). The skilled person will recognise that the lower the $K_D$ value, the less of the compound needed to achieve the desired effect, and as a result, the chances of undesirable

off-target effects are reduced.

Compound affinity was determined by measuring the association and dissociation of compounds to Human (6-His)Sortilin (RnD Systems) using a Creoptix Wave instrument (Creoptix - Malvern Panalytical). Both Human (6-His)Sortilin immobilisation and compound evaluation were performed in HBS-N running buffer: 10 mM HEPES pH 7.4, 150 mM NaCl, 1 % DMSO - Cytiva which was filtered and degassed for 15 minutes before use.

A 4PCH WAVEchip (Creoptix - Malvern Panalytical) was conditioned prior to immobilisiation using 0.2x HBS-N running buffer with 0.1 M Borate pH 9.0, 1 M NaCl (Xantec Bioanalytics) injected across all flow cells. A buffer exchange was performed to 1x HBS-N running buffer and Human (6-His)Sortilin was immobililised in pH 5.0 Acetate solution (Cytiva) via amine coupling following EDC/NHS (Cytiva) surface activation and then passivated via injection of 50 mM Tris pH 7.4.

Compounds were tested in 'WAVE-RAPID' assays at a single concentration (usually: 1 $\mu$M, 10 $\mu$M or 100 $\mu$M) in time-response format (short pulses of increasing duration) using either 'Weak-Binders' (Settings: 400 $\mu$l/min flow rate, 40 Hz acquisiton rate, 5 seconds association time and 20 seconds dissociation time) or 'Intermediate Binders' (Settings: 100 $\mu$l/min flow rate, 10 Hz acquisiton rate, 25 seconds association time and 300 seconds dissociation time) settings. Which was deemed most appropriate was based upon the affinity and kinetics of the compound tested.

DMSO calibration was performed as per manufacturers requirements by injection of running buffer containing DMSO 0.5% above that of the running buffer DMSO (1.5 % in that instance) every 20 cycles. All data was double referenced (blank and reference-channel subtracted) with $K_D$ values calculated by fitting to a '1:1 kinetic' binding model using 'WAVEcontrol' software (Creoptix - Malvern Panalytical).

The data in Table 1 below shows that the compounds of the invention are sortilin binders and inhibitors.

**Table 1**

| Example | h-Sortilin GCI $K_D$ ($\mu$M) | $IC_{50}$ [3H]Neurotensin SPA ($\mu$M) |
|---|---|---|
| 1 | 0.304 | 0.74 |
| 2 | 0.079 | 0.44 |
| 4 | - | 1.07 |
| 5 | 0.713 | - |
| 6 | 0.760 | - |
| 7 | 0.509 | - |
| 8 | 0.587 | - |

**REFERENCES**

Andersen, J et al., Identification of the first small-molecule ligand of the neuronal receptor sortilin and structure determination of the receptor-ligand complex. Acta Crystallogr D Biol Crystallogr (2014), 70(Pt 2), pp.451-460;

Baker, M.et al., Mutations in progranulin cause tau-negative frontotemporal dementia linked to chromosome 17. Nature (2006), 442(7105), pp. 916-919;

Brouwers, N.et al., Genetic variability in progranulin contributes to risk for clinically diagnosed Alzheimer disease. Neurology, (2008), 71(9), pp. 656-664;

Buttenshon, H.N. et al., Increased serum levels of sortilin are associated with depression and correlated with BDNF and VEGF, Nature Translational Psychiatry (2015), 5(e677), pp. 1-7;

Carecchio, M.,et al., Cerebrospinal fluid biomarkers in Progranulin mutations carriers. J Alzheimers Dis (2011), 27(4), pp. 781-790;

Carrasquillo, M. et al.,. Genome-wide screen identifies rs646776 near sortilin as a regulator of progranulin levels in human plasma. Am J Hum Genet (2010), 87(6), pp. 890-897;

Chen, Z. Y.et al., Sortilin controls intracellular sorting of brain-derived neurotrophic factor to the regulated secretory pathway. J Neurosci (2005), 25(26), pp. 6156-6166;

Cruts, M. et al., Loss of progranulin function in frontotemporal lobar degeneration. Trends Genet (2008), 24(4), pp. 186-194;

De Muynck, L. et al., The neurotrophic properties of progranulin depend on the granulin E domain but do not require sortilin binding. Neurobiol Aging (2013), 34(11), pp. 2541-2547;

Egashira, Y. et al.,The growth factor progranulin attenuates neuronal injury induced by cerebral ischemia-reperfusion through the suppression of neutrophil recruitment. J Neuroinflammation (2013), 10, pp. 105;

Galimberti, D. et al.,. GRN variability contributes to sporadic frontotemporal lobar degeneration. J Alzheimers Dis (2010), 19(1), pp. 171-177;

Galimberti, D.et al.,. Progranulin as a therapeutic target for dementia. Expert Opin Ther Targets (2018), 22(7), pp. 579-585. doi:10.1080/14728222.2018.1487951

Gao, A. et al., Implications of Sortilin in Lipid Metabolism and Lipid Disorder Diseases. DNA and Cell Biology (2017), 36(12), pp.1050-1061;

Gass, J. et al., Progranulin regulates neuronal outgrowth independent of sortilin. Mol Neurodegener (2012), 7, pp. 33;

Gass, J. et al., Progranulin: an emerging target for FTLD therapies. Brain Res (2012), 1462, pp. 118-128;

Gijselinck, I.,et al., Granulin mutations associated with frontotemporal lobar degeneration and related disorders: an update. Hum Mutat (2008), 29(12), pp. 1373-1386;

Goettsch, C., et al., Sortilin and Its Multiple Roles in Cardiovascular and Metabolic Diseases. Atherosclerosis, Thrombosis and Vascular Biology (2017), 38(1), pp. 19-25

Jansen, P., et al., Roles for the pro-neurotrophin receptor sortilin in neuronal development, aging and brain injury. Nature Neuroscience (2007), 10(11), pp.1449-1457;

Hu, F. et al., Sortilin-mediated endocytosis determines levels of the frontotemporal dementia protein, progranulin. Neuron (2010), 68(4), pp. 654-667;

Huang, G. et al., Insulin responsiveness of glucose transporter 4 in 3T3-L1 cells depends on the presence of sortilin. Mol Biol Cell (2013), 24(19), pp.3115-3122;

Kaddai, V. et al. Involvement of TNF-$\alpha$ in abnormal adipocyte and muscle sortilin expression in obese mice and humans. Diabetologia (2009) 52, pp. 932-940;

Kjolby, M.et al., Sort1, encoded by the cardiovascular risk locus 1p13.3, is a regulator of hepatic lipoprotein export. Cell Metab (2010), 12(3), pp. 213-223;

Laird, A. S. et al., Progranulin is neurotrophic in vivo and protects against a mutant TDP-43 induced axonopathy. PLoS One (2010), 5(10), e13368;

Lee, W. et al., Targeted manipulation of the sortilin-progranulin axis rescues progranulin haploinsufficiency. Hum Mol Genet (2014), 23(6), pp. 1467-1478;

Martens, L.et al., Progranulin deficiency promotes neuroinflammation and neuron loss following toxin-induced injury. J Clin Invest (2012), 122(11), pp. 3955-3959;

Mazella, J. et al., The 100-kDa neurotensin receptor is gp95/sortilin, a non-G-protein-coupled receptor. J Biol Chem (1998), 273(41), pp. 26273-26276;

Miyakawa, S. et al, Anti-sortilin1 Antibody Up-Regulates Progranulin via Sortilin1 Down-Regulation. Front Neurosci (2020), 14, pp. 586107;

Møller et al. Sortilin as a Biomarker for Cardiovascular Disease Revisited. Frontiers in Cardiovascular Medicine (2021), 8, 652584;

Mortensen, M.B. et al., Targeting sortilin in immune cells reduces proinflammatory cytokines and atherosclerosis. J Clin Invest (2014), 124(12), pp. 5317-5322;

Nykjaer, A et al., Sortilin is essential for proNGF-induced neuronal cell death. Nature (2014), 427(6977), pp. 843-848;

Nykjaer, A., & Willnow, T. E, Sortilin: a receptor to regulate neuronal viability and function. Trends Neurosci (2012), 35(4), pp. 261-270.

Oh, T.J. et al., Circulating sortilin level as a potential biomarker for coronary atherosclerosis and diabetes mellitus. Cardiovascular Diabetology (2017), 16(92);

Pan, X. et al., Sortilin and retromer mediate retrograde transport of Glut4 in 3T3-L1 adipocytes. Mol Biol Cell (2017), 28(12), pp.1667-1675;

Petersen, C. et al., Molecular identification of a novel candidate sorting receptor purified from human brain by receptor-associated protein affinity chromatography. J Biol Chem (1997), 272(6), pp. 3599-3605;

Pickford, F.et al., Progranulin is a chemoattractant for microglia and stimulates their endocytic activity. Am J Pathol (2011), 178(1), pp. 284-295;

Pottier, C., et al., Potential genetic modifiers of disease risk and age at onset in patients with frontotemporal lobar degeneration and GRN mutations: a genome-wide association study. Lancet Neurol (2018), 17(6), pp. 548-558;

Quistgaard, E. et al., Ligands bind to Sortilin in the tunnel of a ten-bladed beta-propeller domain. Nat Struct Mol Biol (2009), 16(1), pp. 96-98;

Santos, A. M. et al., Sortilin Participates in Light-dependent Photoreceptor Degeneration in Vivo. PLoS ONE (2012), 7(4), pp. e36243-e36243.16. Kuruvilla, R. et al., A neurotrophin signaling cascade coordinates sympathetic neuron development through differential control of TrkA trafficking and retrograde signalling. Cell (2004), 118(2), pp. 243-255;

Shi, J. & Kandror, K. V., Sortilin Is Essential and Sufficient for the Formation of Glut4 Storage Vesicles in 3T3-L1 Adipocytes. Developmental Cell (2005), 9, pp. 99-108;

Schroder, T. et al., The identification of AF38469: an orally bioavailable inhibitor of the VPS10P family sorting receptor Sortilin. Bioorg Med Chem Lett (2014), 24(1), pp. 177-180;
Sheng, J. et al.,Progranulin polymorphism rs5848 is associated with increased risk of Alzheimer's disease. Gene (2014), 542(2), pp. 141-145;Skeldal, S. et al., Mapping of the Interaction Site between Sortilin and the p75 Neurotrophin Receptor Reveals a Regulatory Role for the Sortilin Intracellular Domain in p75 Neurotrophin Receptor Shedding and Apoptosis. J Biol Chem (2012), 21(287), pp. 43798-43809;
Tauris, J., et al., Proneurotrophin-3 May Induce Sortilin-Dependent Death In Inner Ear Neurons. Eur J Neuroscience (2020), 33(4), pp.622-31;
Tang, W. et al., The growth factor progranulin binds to TNF receptors and is therapeutic against inflammatory arthritis in mice. Science (2011), 332(6028), pp. 478-484;
Tao, J.et al., Neuroprotective effects of progranulin in ischemic mice. Brain Res (2012), 1436, pp. 130-136;
Tenk, H.K., et al., ProBDNF induces neuronal apoptosis via activation of a receptor complex of p75NTR and sortilin. J Neuroscience (2005), 10(11), pp. 1449-1457
Van Kampen, J. M.,et al., Progranulin gene delivery protects dopaminergic neurons in a mouse model of Parkinson's disease. PLoS One (2014), 9(5), e97032;
Willnow, T. E.et al., VPS10P-domain receptors - regulators of neuronal viability and function. Nat Rev Neurosci (2008), 9(12), pp. 899-909;
Willnow, T.E., et al., Sortilins: new players in lipoprotein metabolism. Current Opinion in Lipidology (2011), 22(2), pp. 79-85.
Wuts, P.G.M. and Greene, T.W, Greene's Protective Groups in Organic Synthesis, 4th Edition, John Wiley and Sons, New York (2006);
Xu, S.H. et al., Regional and Cellular Mapping of Sortilin Immunoreactivity in Adult Human Brain, Frotiers in Neuroanatomy (2019), 13(31), pp. 1-27;
Yano, H., et al., Proneurotrophin-3 is a neuronal apoptotic ligand: evidence for retrograde-directed cell killing. J Neurosci (2009), 29(47), pp. 14790-14802;
Yin, F., et al., Exaggerated inflammation, impaired host defense, and neuropathology in progranulin-deficient mice. J Exp Med (2010), 207(1), pp. 117-128;
Zheng, Y., et al., C-terminus of progranulin interacts with the beta-propeller region of sortilin to regulate progranulin trafficking. PLoS One (2011), 6(6), e21023;
Zhou, X. et al., Prosaposin facilitates sortilin-independent lysosomal trafficking of progranulin. J Cell Biol (2015), 210(6), pp. 991-1002;
Meneses et al., TDP-43 Pathology in Alzheimer's Disease, Mol Neurodegeneration (2021), 16, 84;
Prudencio et al., Misregulation of human sortilin splicing leads to the generation of a nonfunctional progranulin receptor, Proc Natl Acad Sci U S A (2012), 109(52): 21510-21515;
Beel et al., Progranulin reduces insoluble TDP-43 levels, slows down axonal degeneration and prolongs survival in mutant TDP-43 mice, Mol Neurodegener. (2018), 13: 55.

**Sequences referenced throughout the specification and forming part of the description**

SEQ ID NO: 1 (full length sortilin- isoform 1)

1 MERPWGAADG LSRWPHGLGL LLLLQLLPPS TLSQDRLDAP PPPAAPLPRW
51 SGPIGVSWGL RAAAAGGAFP RGGRWRRSAP GEDEECGRVR DFVAKLANNT
101 HQHVFDDLRG SVSLSWVGDS TGVILVLTTF HVPLVIMTFG QSKLYRSEDY
151 GKNFKDITDL INNTFIRTEF GMAIGPENSG KVVLTAEVSG GSRGGRIFRS
201 SDFAKNFVQT DLPFHPLTQM MYSPQNSDYL LALSTENGLW VSKNFGGKWE
251 EIHKAVCLAK WGSDNTIFFT TYANGSCKAD LGALELWRTS DLGKSFKTIG
301 VKIYSFGLGG RFLFASVMAD KDTTRRIHVS TDQGDTWSMA QLPSVGQEQF
351 YSILAANDDM VFMHVDEPGD TGFGTIFTSD DRGIVYSKSL DRHLYTTTGG
401 ETDFTNVTSL RGVYITSVLS EDNSIQTMIT FDQGGRWTHL RKPENSECDA
451 TAKNKNECSL HIHASYSISQ KLNVPMAPLS EPNAVGIVIA HGSVGDAISV
501 MVPDVYISDD GGYSWTKMLE GPHYYTILDS GGIIVAIEHS SRPINVIKFS
551 TDEGQCWQTY TFTRDPIYFT GLASEPGARS MNISIWGFTE SFLTSQWVSY
601 TIDFKDILER NCEEKDYTIW LAHSTDPEDY EDGCILGYKE QFLRLRKSSM
651 CQNGRDYVVT KQPSICLCSL EDFLCDFGYY RPENDSKCVE QPELKGHDLE
701 FCLYGREEHL TTNGYRKIPG DKCQGGVNPV REVKDLKKKC TSNFLSPEKQ
751 NSKSNSVPII LAIVGLMLVT VVAGVLIVKK YVCGGRFLVH RYSVLQQHAE
801 ANGVDGVDAL DTASHTNKSG YHDDSDEDLL E

SEQ ID NO: 2 (full length sortilin- isoform 2)

1 MERPWGAADG LSRWPHGLGL LLLLQLLPPS TLSQDRLDAP PPPAAPLPRW
51 SGPIGVSWGL RAAAAGGAFP RGGRWRRSAP GEDEECGRVR DFVAKLANNT
101 HQHVFDDLRG SVSLSWVGDS TGVILVLTTF HVPLVIMTFG QSKLYRSEDY
151 GKNFKDITDL INNTFIRTEF GMAIGPENSG KVVLTAEVSG GSRGGRIFRS
201 SDFAKNFVQT DLPFHPLTQM MYSPQNSDYL LALSTENGLW VSKNFGGKWE
251 EIHKAVCLAK WGSDNTIFFT TYANGSCTDL GALELWRTSD LGKSFKTIGV
301 KIYSFGLGGR FLFASVMADK DTTRRIHVST DQGDTWSMAQ LPSVGQEQFY
351 SILAANDDMV FMHVDEPGDT GFGTIFTSDD RGIVYSKSLD RHLYTTTGGE
401 TDFTNVTSLR GVYITSVLSE DNSIQTMITF DQGGRWTHLR KPENSECDAT
451 AKNKNECSLH IHASYSISQK LNVPMAPLSE PNAVGIVIAH GSVGDAISVM
501 VPDVYISDDG GYSWTKMLEG PHYYTILDSG GIIVAIEHSS RPINVIKFST
551 DEGQCWQTYT FTRDPIYFTG LASEPGARSM NISIWGFTES FLTSQWVSYT

601 IDFKDILERN CEEKDYTIWL AHSTDPEDYE DGCILGYKEQ FLRLRKSSVC
651 QNGRDYVVTK QPSICLCSLE DFLCDFGYYR PENDSKCVEQ PELKGHDLEF
701 CLYGREEHLT TNGYRKIPGD KCQGGVNPVR EVKDLKKKCT SNFLSPEKQN
751 SKSNSVPIIL AIVGLMLVTV VAGVLIVKKY VCGGRFLVHR YSVLQQHAEA
801 NGVDGVDALD TASHTNKSGY HDDSDEDLLE

SEQ ID NO: 3 (mature sortilin)

1 MTFGQSKLYR SEDYGKNFKD ITDLINNTFI RTEFGMAIGP ENSGKVVLTA
51 EVSGGSRGGR IFRSSDFAKN FVQTDLPFHP LTQMMYSPQN SDYLLALSTE
101 NGLWVSKNFG GKWEEIHKAV CLAKWGSDNT IFFTTYANGS CTDLGALELW
151 RTSDLGKSFK TIGVKIYSFG LGGRFLFASV MADKDTTRRI HVSTDQGDTW
201 SMAQLPSVGQ EQFYSILAAN DDMVFMHVDE PGDTGFGTIF TSDDRGIVYS
251 KSLDRHLYTT TGGETDFTNV TSLRGVYITS VLSEDNSIQT MITFDQGGRW
301 THLRKPENSE CDATAKNKNE CSLHIHASYS ISQKLNVPMA PLSEPNAVGI
361 VIAHGSVGDA ISVMVPDVYI SDDGGYSWTK MLEGPHYYTI LDSGGIIVAI
401 EHSSRPINVI KFSTDEGQCW QTYTFTRDPI YFTGLASEPG ARSMNISIWG
451 FTESFLTSQW VSYTIDFKDI LERNCEEKDY TIWLAHSTDP EDYEDGCILG
501 YKEQFLRLRK SSVCQNGRDY VVTKQPSICL CSLEDFLCDF GYYRPENDSK
551 CVEQPELKGH DLEFCLYGRE EHLTTNGYRK IPGDKCQGGV NPVREVKDLK
601 KKCTSNFLSP EKQNSKSNSV PIILAIVGLM LVTVVAGVLI VKKYVCGGRF
651 LVHRYSVLQQ HAEANGVDGV DALDTASHTN KSGYHDDSDE DLLE

SEQ ID NO: 4 (Murine Sortilin)

>sp|Q6PHU5|SORT_MOUSE Sortilin OS=Mus musculus OX=10090 GN=Sort1 PE=1 SV=1

MERPRGAADGLLRWPLGLLLLLQLLPPAAVGQDRLDAPPPPAPPLLRWAGPVGVSWGLRA

AAPGGPVPRAGRWRRGAPAEDQDCGRLPDFIAKLTNNTHQHVFDDLSGSVSLSWVGDST
G

VILVLTTFQVPLVIVSFGQSKLYRSEDYGKNFKDITNLINNTFIRTEFGMAIGPENSGKV

ILTAEVSGGSRGGRVFRSSDFAKNFVQTDLPFHPLTQMMYSPQNSDYLLALSTENGLWVS

KNFGEKWEEIHKAVCLAKWGPNNIIFFTTHVNGSCKADLGALELWRTSDLGKTFKTIGVK

IYSFGLGGRFLFASVMADKDTTRRIHVSTDQGDTWSMAQLPSVGQEQFYSILAANEDMVF

MHVDEPGDTGFGTIFTSDDRGIVYSKSLDRHLYTTTGGETDFTNVTSLRGVYITSTLSED

NSIQSMITFDQGGRWEHLRKPENSKCDATAKNKNECSLHIHASYSISQKLNVPMAPLSEP

NAVGIVIAHGSVGDAISVMVPDVYISDDGGYSWAKMLEGPHYYTILDSGGIIVAIEHSNR

PINVIKFSTDEGQCWQSYVFTQEPIYFTGLASEPGARSMNISIWGFTESFITRQWVSYTV

DFKDILERNCEEDDYTTWLAHSTDPGDYKDGCILGYKEQFLRLRKSSVCQNGRDYVVAKQ

PSVCPCSLEDFLCDFGYFRPENASECVEQPELKGHELEFCLYGKEEHLTTNGYRKIPGDK

CQGGMNPAREVKDLKKKCTSNFLNPTKQNSKSNSVPIILAIVGLMLVTVVAGVLIVKKYV

CGGRFLVHRYSVLQQHAEADGVEALDSTSHAKSGYHDDSDEDLLE

**Claims**

**1.** A compound of formula (I)

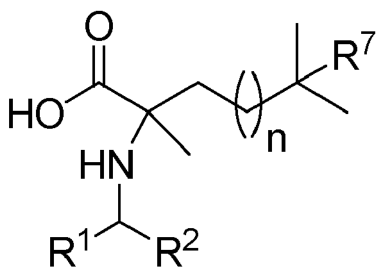

(I)

or a pharmaceutically acceptable salt, solvate, hydrate, tautomer, optical isomer, N-oxide, and/or prodrug thereof; wherein

$R^1$ is

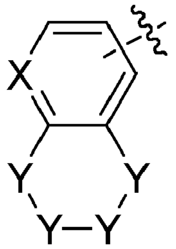

or

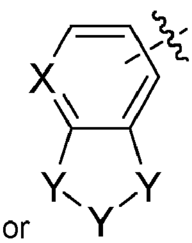

;

X is $CR^3$ or N, wherein $R^3$ is selected from the group consisting of H, halo, and C1-C4 alkyl;
each Y is independently $CHR^4$, $NR^5$, $CR^6R^6$, or O, wherein $R^4$ is independently selected from the group consisting of H, halo, oxo, and $C_1$-$C_4$ alkyl; $R^5$ is independently selected from the group consisting of H, halo, $C_1$-$C_4$ alkyl, $C_1$-$C_4$ hydroxyalkyl, -($C_1$-$C_4$ alkyl)-O-($C_1$-$C_4$ alkyl), -C(O)-($C_1$-$C_4$ alkyl), and -C(O)O-($C_1$-$C_4$ alkyl); and $R^6$ is

independently selected from the group consisting of halo and $C_1$-$C_4$ alkyl;
$R^2$ is selected from the group consisting of: H, $C_1$-$C_4$ alkyl, $C_1$-$C_4$ hydroxyalkyl and $C_1$-$C_4$ haloalkyl;
$R^7$ is H or $-CH_3$; and
n = 0 or 1.

**2.** The compound according to claim 1, wherein $R^2$ is selected from the group consisting of H, $CH_3$, $CH_2F$, $CHF_2$, and $CF_3$, preferably wherein $R^2$ is selected from the group consisting of H, $CH_3$, $CHF_2$, and $CF_3$, more preferably H.

**3.** The compound according to claim 1 or 2, wherein each Y is independently $CHR^4$, $NR^5$, or O; and/or

wherein $R^3$ is H or $C_1$-$C_4$ alkyl, preferably H or methyl; and/or
wherein each $R^4$ is independently H or oxo; and/or
wherein each $R^5$ is independently H, $C_1$-$C_4$ alkyl, or-C(O)O-($C_1$-$C_4$ alkyl), preferably H, methyl, or -C(O)O-(tert-butyl), more preferably H or methyl; and/or
wherein $R^7$ is $-CH_3$; and/or
wherein n = 1.

**4.** The compound according to any preceding claim, wherein $R^1$ is selected from one of the following groups:

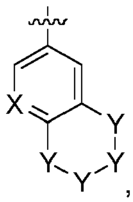, 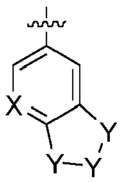, 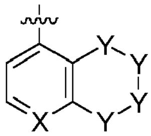, and 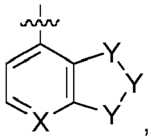,

preferably wherein no more than one or two Y are $NR^5$ or O and the remaining Y are $CHR^4$.

**5.** The compound according to claim 4, wherein $R^1$ is selected from one of the following groups:

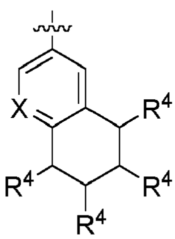, 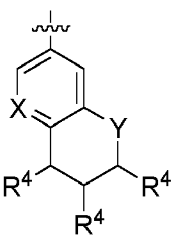, 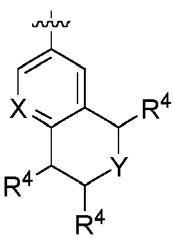, 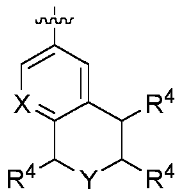, 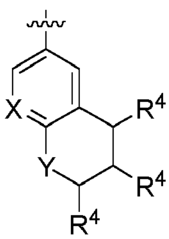, 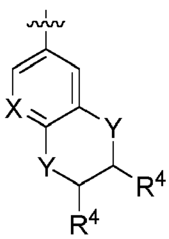,

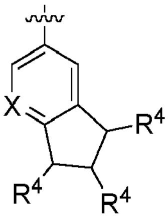, 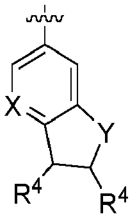, 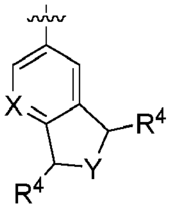, 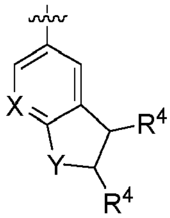, 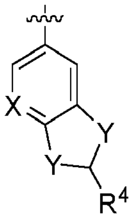, 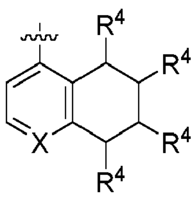,

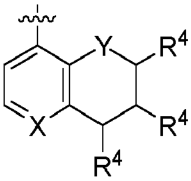, 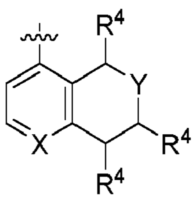, 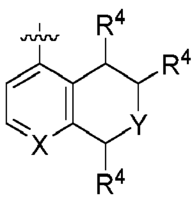, 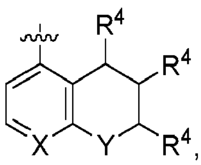, 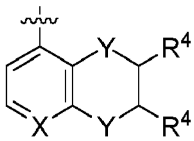,

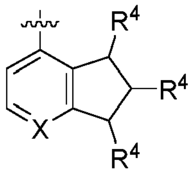, 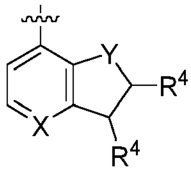, 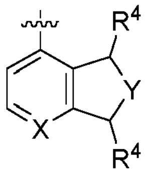, 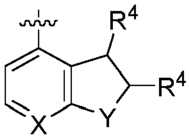, and 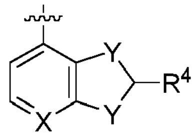,

wherein each Y is independently $NR^5$ or O, preferably wherein one $R^4$ group is H or oxo and the other $R^4$ groups are all H.

**6.** The compound according to claim 5, wherein $R^1$ is selected from one of the following groups:

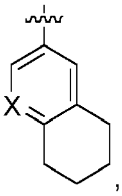, 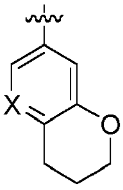, 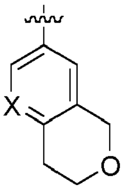, 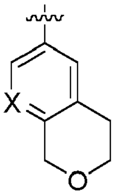, 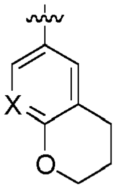, 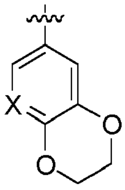, 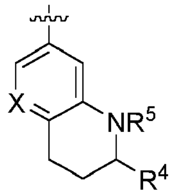,

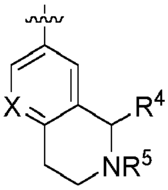, 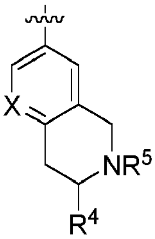, 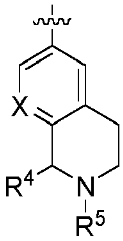, 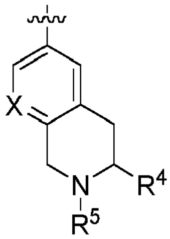, 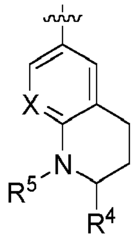, 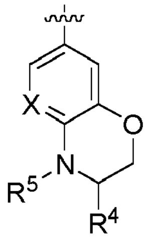,

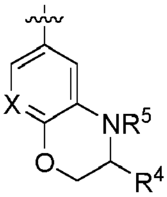, 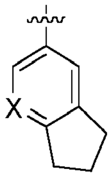, 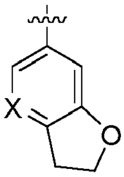, 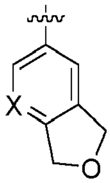, 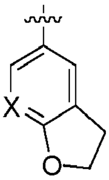, 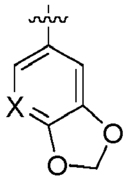, 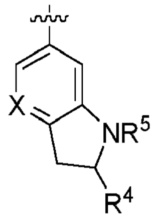,

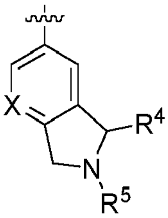, 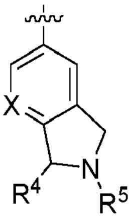, 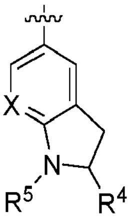, 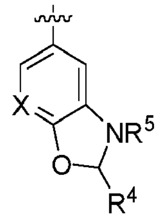, 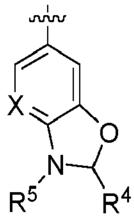, 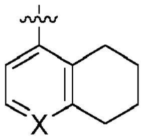, 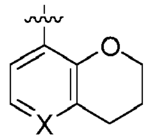,

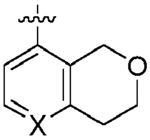, 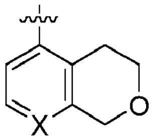, 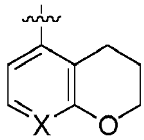, 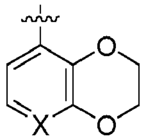, 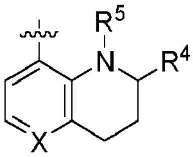, 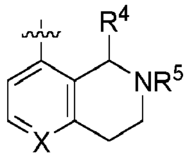,

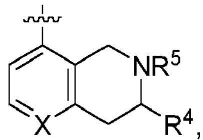, 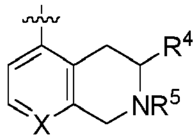, 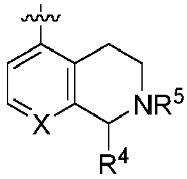, 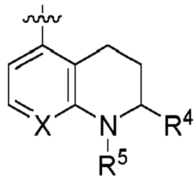, 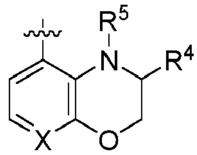,

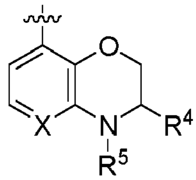, 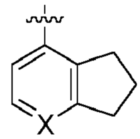, 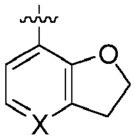, 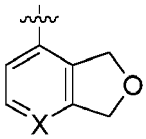, 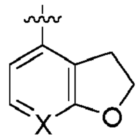, 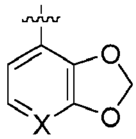,

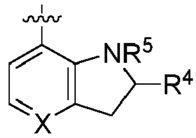, 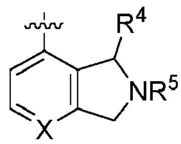, 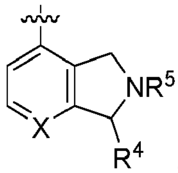, 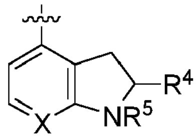, 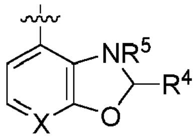, and

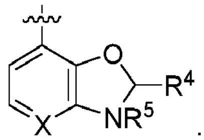.

**7.** The compound according to claim 6, wherein $R^1$ is selected from one of the following groups:

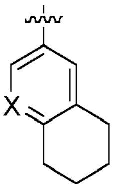, 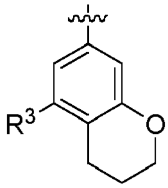, 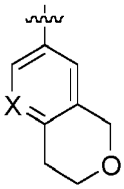, 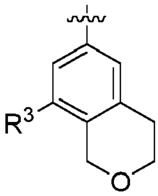, 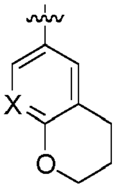, 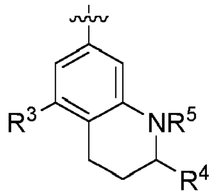,

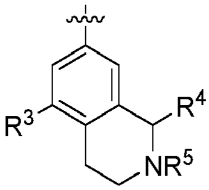, 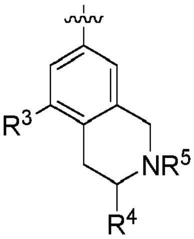, 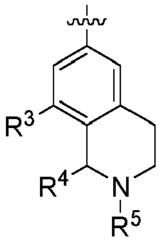, 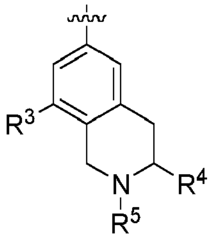, 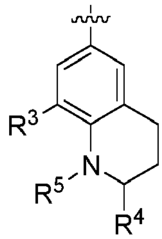, 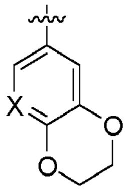

, 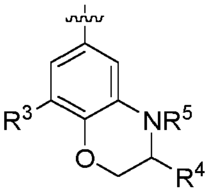, 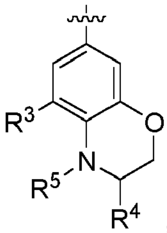, 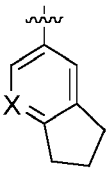, 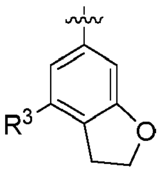, 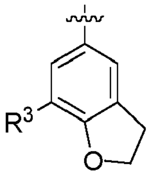, 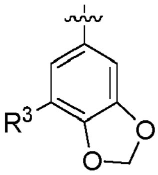,

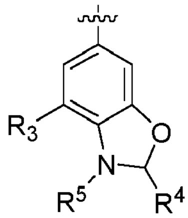, 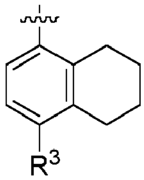, 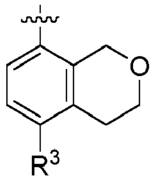, 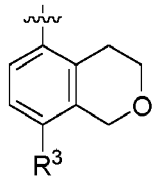, 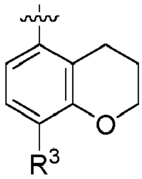, 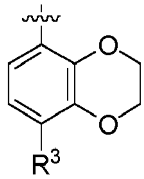,

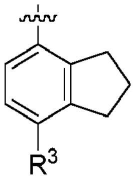, 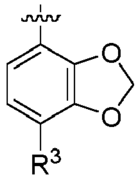, and 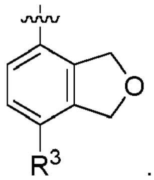.

**8.** The compound according to claim 7, wherein $R^1$ is selected from one of the following groups:

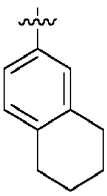, 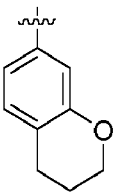, 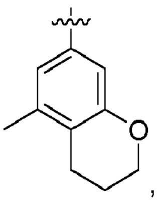, 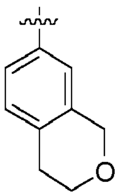, 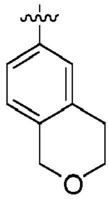, 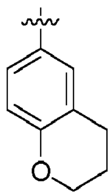, 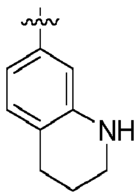, 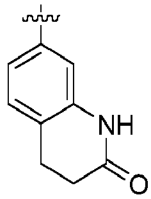,

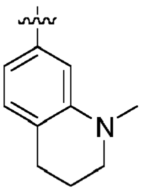, 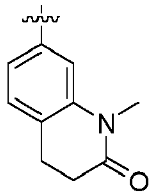, 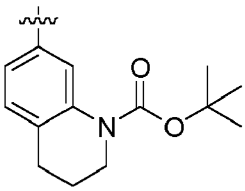, 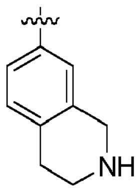, 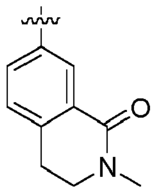, 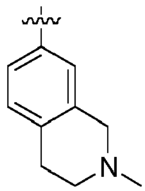,

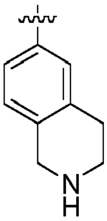, 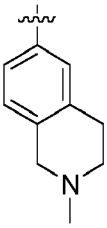, 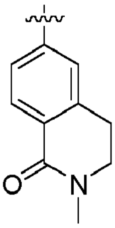, 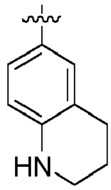, 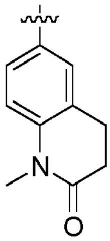, 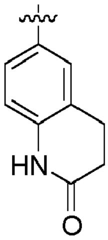, 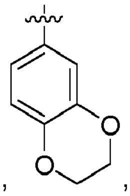, 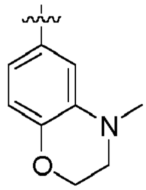,

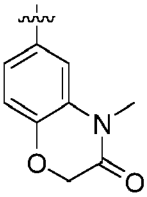, 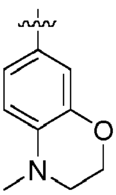, 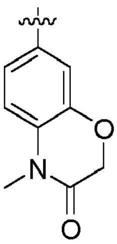, 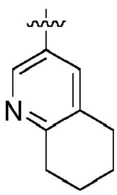, 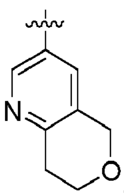, 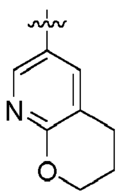, 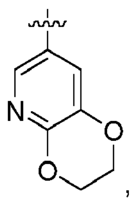, 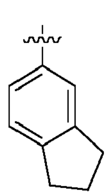,

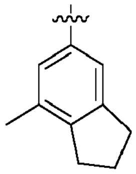, 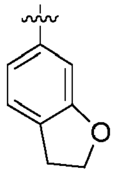, 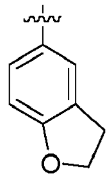, 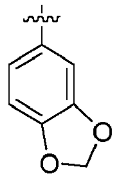, 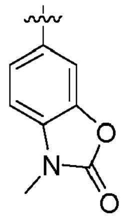, 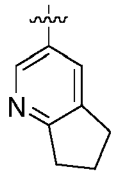, 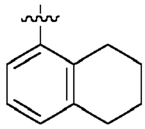, 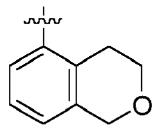,

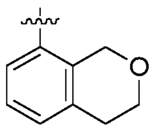, 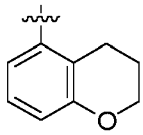, 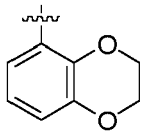, 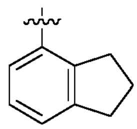, 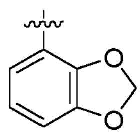, and 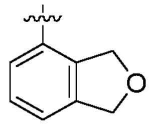,

preferably $R^1$ is selected from one of the following groups:

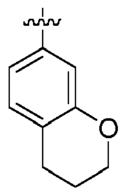, 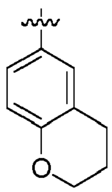, 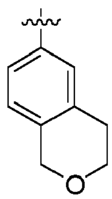, 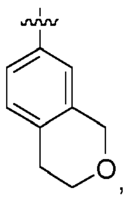, 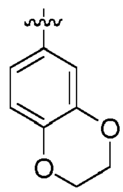, 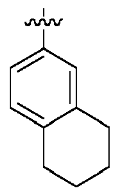, 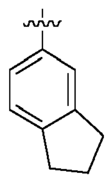, and 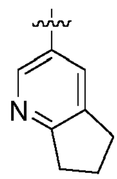.

**9.** The compound according to any preceding claim, wherein the compound is:

2,5,5-trimethyl-2-{[(5,6,7,8-tetrahydro-2-naphthyl)methyl]amino}hexanoic acid;
(S)-2,5,5-trimethyl-2-{[(5,6,7,8-tetrahydro-2-naphthyl)methyl]amino}hexanoic acid;
(R)-2,5,5-trimethyl-2-{[(5,6,7,8-tetrahydro-2-naphthyl)methyl]amino}hexanoic acid;
2-{[(5-indanyl)methyl]amino}-2,5,5-trimethylhexanoic acid;
2-{[(7-chromanyl)methyl]amino}-2,5,5-trimethylhexanoic acid;
2-{[(6-chromanyl)methyl]amino}-2,5,5-trimethylhexanoic acid;
2-{[(6-isochromanyl)methyl]amino}-2,5,5-trimethylhexanoic acid;
2-{[(7-isochromanyl)methyl]amino}-2,5,5-trimethylhexanoic acid;
(R)-2-{[(5-indanyl)methyl]amino}-2,5,5-trimethylhexanoic acid;
(S)-2-{[(5-indanyl)methyl]amino}-2,5,5-trimethylhexanoic acid;
(S)-2-{[(4-aza-6-indanyl)methyl]amino}-2,5,5-trimethylhexanoic acid;
(S)-2-{[(2,3-dihydro-1,4-benzodioxin-6-yl)methyl]amino}-2,5,5-trimethylhexanoic acid;
(S)-2-{[(6-chromanyl)methyl]amino}-2,5,5-trimethylhexanoic acid;
(S)-2-{[(6-isochromanyl)methyl]amino}-2,5,5-trimethylhexanoic acid;
(S)-2-{[(7-isochromanyl)methyl]amino}-2,5,5-trimethylhexanoic acid;
(S)-2-{[(7-chromanyl)methyl]amino}-2,5,5-trimethylhexanoic acid;

or a pharmaceutically acceptable salt, solvate, hydrate, tautomer, optical isomer, N-oxide, and/or prodrug thereof.

**10.** A pharmaceutical composition comprising a compound according to any preceding claim and a pharmaceutically acceptable carrier, excipient, and/or diluent.

**11.** The compound according to any one of claims 1 to 9, or the pharmaceutical composition of claim 10, for use in therapy.

**12.** The compound according to any one of claims 1 to 9, or the pharmaceutical composition of claim 10, for use in the treatment or prevention of a neurodegenerative disorder, a psychiatric disorder, an inflammatory disorder, a lysosomal storage disorder, a cancer, pain, diabetes mellitus, retinopathies such as diabetic retinopathy, brain tumours, glaucoma, uveitis, cardiovascular diseases, kidney disease, psoriasis, hereditary eye conditions, chronic pain, hearing loss or diseases **characterized by** misfolded tau;

preferably wherein the neurodegenerative disorder is selected from motor neuron diseases, Frontotemporal

Lobar Degeneration (FTLD), frontotemporal dementia, Alzheimer's disease, Parkinson's disease, Huntington's disease, prion diseases such as Creutzfeldt-Jakob disease (CJD), acute brain injury, spinal cord injury and stroke, preferably wherein the motor neuron disease is selected from amyotrophic lateral sclerosis (ALS), Primary Lateral Sclerosis, and Progressive Muscular Atrophy;
preferably wherein the neurodegenerative disorder is **characterised by** misfolded TAR DNA-binding protein 43, such as amyotrophic lateral sclerosis, Alzheimer's disease, Frontotemporal Lobar Degeneration, or frontotemporal dementia;
preferably wherein the psychiatric disorder is selected from bipolar disorder, major depression, post-traumatic stress disorder, and anxiety disorders.
preferably wherein the inflammatory disorder is selected from inflammatory diseases and neuroinflammation;
preferably wherein the lysosomal storage disorder is selected from the group consisting of NCL/Batten Disease caused by mutations in CLN gene *CLN1 (PPT1), CLN2 (TPP1), CLN3, CLN4 (DNAJC5), CLN5, CLN6, CLN7 (MFSD8), CLN8, CLN10 (CTSD), CLN11, CLN12 (ATP13A2), CLN13 (CTSF), CLN14 (KCTD7), CLCN6,* and/or *SGSH;* Pompe disease, Fabry disease, Gaucher disease, Niemann-Pick disease Types A, B, and C; GM1 gangliosidosis, GM2 gangliosidosis (including Sandhoff and Tay-Sachs), mucopolysachariddoses (MPS) types I (Hurler disease)/II (Hunter disease)/IIIa (Sanfilippo A)/IIIB (Sanfilippo B)/IIIc (Sanfilippo C)/IIId (Sanfilippo D)/IVA (Morqui" A)/'VB/VI/VII (Sly)/IX, mucolipisosis III (I-cell) and IV, multiple sulfatase deficiency; sialidosis, galactosialidosis, $\alpha$-mannosidosis, $\beta$-mannosidosis, apartylglucosaminuria, fucosidosis, Schindler disease, metachromatic leukodystrophy caused by deficiencies in either arylsulfatase A or Saposin B, globoid cell leukodystrophy (Krabbe disease), Farber lipogranulomatosis, Wolman and cholesteryl ester storage disease, pycnodystostosis, cystinosis, Salla disease, Danon disease, Griscelli disease Types ½/3, Hermansky Pudliak Disease, and Chédiak-Higashi syndrome;
preferably wherein the cancer is selected from breast cancer, lung cancer, ovarian cancer, prostate cancer, thyroid cancer, pancreatic cancer, glioblastoma and colorectal cancer;
preferably wherein the cardiovascular disease is selected from atherosclerosis, cardiomyopathy, heart attack, arrhythmias, heart failure, and ischemic heart disease; and
preferably wherein the hearing loss is selected from noise-induced hearing loss, ototoxicity induced hearing loss, age-induced hearing loss, idiopathic hearing loss, tinnitus and sudden hearing loss.

Europäisches Patentamt
European Patent Office
Office européen des brevets

# EUROPEAN SEARCH REPORT

Application Number
EP 23 16 0617

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 3 981 394 A1 (INSUSENSE APS [DK]) 13 April 2022 (2022-04-13) * claim 5; compound (III) * * page 29; compound 63 * ----- | 1-12 | INV. C07D221/04 C07C229/14 C07C309/04 C07D311/58 C07D319/18 A61K31/198 A61K31/353 A61K31/357 A61P9/00 A61P13/12 A61P17/00 A61P25/00 A61P25/28 A61P27/02 A61P29/00 A61P35/00 |
| A,D | ANDERSEN JACOB LAUWRING ET AL: "Identification of the first small-molecule ligand of the neuronal receptor sortilin and structure determination of the receptor-ligand complex", ACTA CRYSTALLOGRAPHICA SECTION D BIOLOGICAL CRYSTALLOGRAPHY, vol. 70, no. 2, 29 January 2014 (2014-01-29), pages 451-460, XP055893559, DOI: 10.1107/S1399004713030149 * page 454; figure 1; compounds 1a,1c,1e,1h, AF-40431 * ----- | 1-12 | |
| A | ANDERSEN JACOB LAUWRING ET AL: "The identification of novel acid isostere based inhibitors of the VPS10P family sorting receptor Sortilin", BIOORGANIC & MEDICINAL CHEMISTRY LETTERS, ELSEVIER, AMSTERDAM NL, vol. 27, no. 11, 20 February 2017 (2017-02-20), pages 2629-2633, XP085002011, ISSN: 0960-894X, DOI: 10.1016/J.BMCL.2017.02.028 * page 2631 - page 2632 * ----- | 1-12 | TECHNICAL FIELDS SEARCHED (IPC) C07D C07C |
| A | WO 2014/114779 A1 (LUNDBECK & CO AS H [DK]) 31 July 2014 (2014-07-31) * claims; examples * ----- -/-- | 1-12 | |

The present search report has been drawn up for all claims

1

| Place of search | Date of completion of the search | Examiner |
|---|---|---|
| Munich | 26 July 2023 | Bedel, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 23 16 0617

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 4 089 102 A1 (INSUSENSE APS [DK]) 16 November 2022 (2022-11-16) * claims; examples * ----- | 1-12 | |
| E | WO 2023/031440 A1 (INSUSENSE APS [DK]) 9 March 2023 (2023-03-09) * claim 3; examples * ----- | 1-12 | |

TECHNICAL FIELDS SEARCHED (IPC)

The present search report has been drawn up for all claims

1

| Place of search | Date of completion of the search | Examiner |
|---|---|---|
| Munich | 26 July 2023 | Bedel, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO.

EP 23 16 0617

This annex lists the patent family members relating to the patent documents cited in the above-mentioned European search report.
The members are as contained in the European Patent Office EDP file on
The European Patent Office is in no way liable for these particulars which are merely given for the purpose of information.

26-07-2023

| Patent document cited in search report | Publication date | Patent family member(s) | Publication date |
|---|---|---|---|
| EP 3981394 A1 | 13-04-2022 | AU 2021356121 A1 | 18-05-2023 |
| | | CA 3194843 A1 | 14-04-2022 |
| | | CN 116194096 A | 30-05-2023 |
| | | EP 3981394 A1 | 13-04-2022 |
| | | EP 4225280 A1 | 16-08-2023 |
| | | KR 20230084174 A | 12-06-2023 |
| | | WO 2022074119 A1 | 14-04-2022 |
| WO 2014114779 A1 | 31-07-2014 | CN 104955456 A | 30-09-2015 |
| | | EP 2948147 A1 | 02-12-2015 |
| | | HK 1217431 A1 | 13-01-2017 |
| | | JP 6409004 B2 | 17-10-2018 |
| | | JP 2016505063 A | 18-02-2016 |
| | | US 2015368231 A1 | 24-12-2015 |
| | | US 2017239226 A1 | 24-08-2017 |
| | | WO 2014114779 A1 | 31-07-2014 |
| EP 4089102 A1 | 16-11-2022 | EP 4089102 A1 | 16-11-2022 |
| | | WO 2022238565 A2 | 17-11-2022 |
| WO 2023031440 A1 | 09-03-2023 | EP 4144352 A1 | 08-03-2023 |
| | | WO 2023031440 A1 | 09-03-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20160331746 A
- WO 2021116290 A1
- EP 22193792


### Non-patent literature cited in the description


- **SILVERMAN, R. B.** The Organic Chemistry of Drug Design and Drug Action. Elsevier Academic Press, 2004, 498-549
- **GREENE ; WUTS.** Protective Groups in Organic Synthesis. John Wiley and Sons, 2006
- **ANDERSEN, J et al.** Identification of the first small-molecule ligand of the neuronal receptor sortilin and structure determination of the receptor-ligand complex. *Acta Crystallogr D Biol Crystallogr,* 2014, vol. 70, 451-460
- **BAKER, M. et al.** Mutations in progranulin cause tau-negative frontotemporal dementia linked to chromosome 17. *Nature,* 2006, vol. 442 (7105), 916-919
- **BROUWERS, N.** Genetic variability in progranulin contributes to risk for clinically diagnosed Alzheimer disease. *Neurology,* 2008, vol. 71 (9), 656-664
- **BUTTENSHON, H.N. et al.** Increased serum levels of sortilin are associated with depression and correlated with BDNF and VEGF. *Nature Translational Psychiatry,* 2015, vol. 5 (e677), 1-7
- **CARECCHIO, M.** Cerebrospinal fluid biomarkers in Progranulin mutations carriers. *J Alzheimers Dis,* 2011, vol. 27 (4), 781-790
- **CARRASQUILLO, M. et al.** Genome-wide screen identifies rs646776 near sortilin as a regulator of progranulin levels in human plasma. *Am J Hum Genet,* 2010, vol. 87 (6), 890-897
- **CHEN, Z. Y. et al.** Sortilin controls intracellular sorting of brain-derived neurotrophic factor to the regulated secretory pathway. *J Neurosci,* 2005, vol. 25 (26), 6156-6166
- **CRUTS, M. et al.** Loss of progranulin function in frontotemporal lobar degeneration. *Trends Genet,* 2008, vol. 24 (4), 186-194
- **DE MUYNCK, L. et al.** The neurotrophic properties of progranulin depend on the granulin E domain but do not require sortilin binding. *Neurobiol Aging,* 2013, vol. 34 (11), 2541-2547
- **EGASHIRA, Y. et al.** The growth factor progranulin attenuates neuronal injury induced by cerebral ischemia-reperfusion through the suppression of neutrophil recruitment. *J Neuroinflammation,* 2013, vol. 10, 105
- **GALIMBERTI, D. et al.** GRN variability contributes to sporadic frontotemporal lobar degeneration. *J Alzheimers Dis,* 2010, vol. 19 (1), 171-177
- **GALIMBERTI, D.** Progranulin as a therapeutic target for dementia. *Expert Opin Ther Targets,* 2018, vol. 22 (7), 579-585
- **GAO, A. et al.** Implications of Sortilin in Lipid Metabolism and Lipid Disorder Diseases. *DNA and Cell Biology,* 2017, vol. 36 (12), 1050-1061
- **GASS, J. et al.** Progranulin regulates neuronal outgrowth independent of sortilin. *Mol Neurodegener,* 2012, vol. 7, 33
- **GASS, J. et al.** Progranulin: an emerging target for FTLD therapies. *Brain Res,* 2012, vol. 1462, 118-128
- **GIJSELINCK, I.** Granulin mutations associated with frontotemporal lobar degeneration and related disorders: an update. *Hum Mutat,* 2008, vol. 29 (12), 1373-1386
- **GOETTSCH, C. et al.** Sortilin and Its Multiple Roles in Cardiovascular and Metabolic Diseases. *Atherosclerosis, Thrombosis and Vascular Biology,* 2017, vol. 38 (1), 19-25
- **JANSEN, P. et al.** Roles for the pro-neurotrophin receptor sortilin in neuronal development, aging and brain injury. *Nature Neuroscience,* 2007, vol. 10 (11), 1449-1457
- **HU, F. et al.** Sortilin-mediated endocytosis determines levels of the frontotemporal dementia protein, progranulin. *Neuron,* 2010, vol. 68 (4), 654-667
- **HUANG, G. et al.** Insulin responsiveness of glucose transporter 4 in 3T3-L1 cells depends on the presence of sortilin. *Mol Biol Cell,* 2013, vol. 24 (19), 3115-3122

- **KADDAI, V. et al.** Involvement of TNF-α in abnormal adipocyte and muscle sortilin expression in obese mice and humans. *Diabetologia,* 2009, vol. 52, 932-940
- **KJOLBY, M. et al.** Sort1, encoded by the cardiovascular risk locus 1p13.3, is a regulator of hepatic lipoprotein export. *Cell Metab,* 2010, vol. 12 (3), 213-223
- **LAIRD, A. S. et al.** Progranulin is neurotrophic in vivo and protects against a mutant TDP-43 induced axonopathy. *PLoS One,* 2010, vol. 5 (10), e13368
- **LEE, W. et al.** Targeted manipulation of the sortilin-progranulin axis rescues progranulin haploinsufficiency. *Hum Mol Genet,* 2014, vol. 23 (6), 1467-1478
- **MARTENS, L. et al.** Progranulin deficiency promotes neuroinflammation and neuron loss following toxin-induced injury. *J Clin Invest,* 2012, vol. 122 (11), 3955-3959
- **MAZELLA, J. et al.** The 100-kDa neurotensin receptor is gp95/sortilin, a non-G-protein-coupled receptor. *J Biol Chem,* 1998, vol. 273 (41), 26273-26276
- **MIYAKAWA, S. et al.** Anti-sortilin1 Antibody Up-Regulates Progranulin via Sortilin1 Down-Regulation. *Front Neurosci,* 2020, vol. 14, 586107
- **MØLLER et al.** Sortilin as a Biomarker for Cardiovascular Disease Revisited. *Frontiers in Cardiovascular Medicine,* 2021, vol. 8, 652584
- **MORTENSEN, M.B. et al.** Targeting sortilin in immune cells reduces proinflammatory cytokines and atherosclerosis. *J Clin Invest,* 2014, vol. 124 (12), 5317-5322
- **NYKJAER, A et al.** Sortilin is essential for proNGF-induced neuronal cell death. *Nature,* 2014, vol. 427 (6977), 843-848
- **NYKJAER, A. ; WILLNOW, T. E.** Sortilin: a receptor to regulate neuronal viability and function. *Trends Neurosci,* 2012, vol. 35 (4), 261-270
- **OH, T.J. et al.** Circulating sortilin level as a potential biomarker for coronary atherosclerosis and diabetes mellitus. *Cardiovascular Diabetology,* 2017, vol. 16 (92
- **PAN, X. et al.** Sortilin and retromer mediate retrograde transport of Glut4 in 3T3-L1 adipocytes. *Mol Biol Cell,* 2017, vol. 28 (12), 1667-1675
- **PETERSEN, C. et al.** Molecular identification of a novel candidate sorting receptor purified from human brain by receptor-associated protein affinity chromatography. *J Biol Chem,* 1997, vol. 272 (6), 3599-3605
- **PICKFORD, F. et al.** Progranulin is a chemoattractant for microglia and stimulates their endocytic activity. *Am J Pathol,* 2011, vol. 178 (1), 284-295
- **POTTIER, C. et al.** Potential genetic modifiers of disease risk and age at onset in patients with frontotemporal lobar degeneration and GRN mutations: a genome-wide association study. *Lancet Neurol,* 2018, vol. 17 (6), 548-558
- **QUISTGAARD, E. et al.** Ligands bind to Sortilin in the tunnel of a ten-bladed beta-propeller domain. *Nat Struct Mol Biol,* 2009, vol. 16 (1), 96-98
- **SANTOS, A. M. et al.** Sortilin Participates in Light-dependent Photoreceptor Degeneration in Vivo. *PLoS ONE,* 2012, vol. 7 (4), e36243-e36243.16
- **KURUVILLA, R. et al.** A neurotrophin signaling cascade coordinates sympathetic neuron development through differential control of TrkA trafficking and retrograde signalling. *Cell,* 2004, vol. 118 (2), 243-255
- **SHI, J. ; KANDROR, K. V.** Sortilin Is Essential and Sufficient for the Formation of Glut4 Storage Vesicles in 3T3-L1 Adipocytes. *Developmental Cell,* 2005, vol. 9, 99-108
- **SCHRODER, T. et al.** The identification of AF38469: an orally bioavailable inhibitor of the VPS10P family sorting receptor Sortilin. *Bioorg Med Chem Lett,* 2014, vol. 24 (1), 177-180
- **SHENG, J. et al.** Progranulin polymorphism rs5848 is associated with increased risk of Alzheimer's disease. *Gene,* 2014, vol. 542 (2), 141-145
- **SKELDAL, S. et al.** Mapping of the Interaction Site between Sortilin and the p75 Neurotrophin Receptor Reveals a Regulatory Role for the Sortilin Intracellular Domain in p75 Neurotrophin Receptor Shedding and Apoptosis. *J Biol Chem,* 2012, vol. 21 (287), 43798-43809
- **TAURIS, J. et al.** Proneurotrophin-3 May Induce Sortilin-Dependent Death In Inner Ear Neurons. *Eur J Neuroscience,* 2020, vol. 33 (4), 622-31
- **TANG, W. et al.** The growth factor progranulin binds to TNF receptors and is therapeutic against inflammatory arthritis in mice. *Science,* 2011, vol. 332 (6028), 478-484
- **TAO, J. et al.** Neuroprotective effects of progranulin in ischemic mice. *Brain Res,* 2012, vol. 1436, 130-136
- **TENK, H.K. et al.** ProBDNF induces neuronal apoptosis via activation of a receptor complex of p75NTR and sortilin. *J Neuroscience,* 2005, vol. 10 (11), 1449-1457
- **VAN KAMPEN, J. M.** Progranulin gene delivery protects dopaminergic neurons in a mouse model of Parkinson's disease. *PLoS One,* 2014, vol. 9 (5), e97032
- **WILLNOW, T. E. et al.** VPS10P-domain receptors - regulators of neuronal viability and function. *Nat Rev Neurosci,* 2008, vol. 9 (12), 899-909
- **WILLNOW, T.E. et al.** Sortilins: new players in lipoprotein metabolism. *Current Opinion in Lipidology,* 2011, vol. 22 (2), 79-85

- **WUTS, P.G.M. ; GREENE, T.W.** Greene's Protective Groups in Organic Synthesis. John Wiley and Sons, 2006
- **XU, S.H. et al.** Regional and Cellular Mapping of Sortilin Immunoreactivity in Adult Human Brain. *Frotiers in Neuroanatomy,* 2019, vol. 13 (31), 1-27
- **YANO, H. et al.** Proneurotrophin-3 is a neuronal apoptotic ligand: evidence for retrograde-directed cell killing. *J Neurosci,* 2009, vol. 29 (47), 14790-14802
- **YIN, F. et al.** Exaggerated inflammation, impaired host defense, and neuropathology in progranulin-deficient mice. *J Exp Med,* 2010, vol. 207 (1), 117-128
- **ZHENG, Y. et al.** C-terminus of progranulin interacts with the beta-propeller region of sortilin to regulate progranulin trafficking. *PLoS One,* 2011, vol. 6 (6), e21023
- **ZHOU, X. et al.** Prosaposin facilitates sortilin-independent lysosomal trafficking of progranulin. *J Cell Biol,* 2015, vol. 210 (6), 991-1002
- **MENESES et al.** TDP-43 Pathology in Alzheimer's Disease. *Mol Neurodegeneration,* 2021, vol. 16, 84
- **PRUDENCIO et al.** Misregulation of human sortilin splicing leads to the generation of a nonfunctional progranulin receptor. *Proc Natl Acad Sci U S A,* 2012, vol. 109 (52), 21510-21515
- **BEEL et al.** Progranulin reduces insoluble TDP-43 levels, slows down axonal degeneration and prolongs survival in mutant TDP-43 mice. *Mol Neurodegener,* 2018, vol. 13, 55